# EUROPEAN PATENT APPLICATION

(11) **EP 3 805 256 A1**
(43) Date of publication of application: **14.04.2021**
(21) Application number: 19810034.9
(22) Date of filing: 28.05.2019
(51) Int. Cl.: C07K 14/47, C07K 1/14, G01N 33/535, C12N 9/10

(54) **LINEAR POLYFUNCTIONAL MULTIMER BIOMOLECULE COUPLED TO POLYUBIQUITIN LINKER AND USE THEREOF**

(30) Priority: 28.05.2018 KR 20180060502
(71) Applicant: Onegene Biotechnology Inc., Gyeonggi-do 16229 (KR)
(72) Inventor: PARK, Sungjin, Seongnam-si, Gyeonggi-do 13540 (KR); IM, Dae Seong, Yongin-si, Gyeonggi-do 17084 (KR); CHOI, Jaeyoung, Suwon-si, Gyeonggi-do 16672 (KR)
(74) Representative: Callaghan, Dayle Anne
(86) International application number: PCT/KR2019/006376
(87) International publication number: WO 2019/231208

(57) **Abstract**

The present invention provides a linear multimeric biomolecule polymer wherein a biomolecule is bonded to a polyubiquitin scaffold formed of two or more covalently bonded ubiquitins, by obtaining, from a host cell, a biomolecule bonded with a ubiquitin C-terminal tag through recombinant expression, and polyubiquitinating the biomolecule in vitro in the presence of proteins involved in ubiquitination, E1 (activation enzyme), E2 (conjugation enzyme), and E3 (ligase), and a substrate. The polymer according to the present invention may be used in the separation and purification of a biomolecule, the separation of a target material that binds to the biomolecule, etc.

## Description

### Technical Field

The present invention relates to a method for preparing a biomolecule including a protein into a polymer in a multimeric form. Specifically, the present invention relates to a method for preparing a biomolecule recombinantly expressed from a host cell into a linear polyfunctional multimeric biomolecule polymer using a ubiquitination system.

### Background Art

Preparing biomolecules and/or small molecule chemical compounds including proteins, peptides, polypeptides, antibodies, DNA and RNA in multimeric form has various advantages. For example, the physicochemical properties of protein such as solubility, gelation, thermal stability and pH stability can be improved by linking two or more homogeneous or heterogeneous proteins using a fusion or a cross linker (or a cross-linking agent). For example, CLEA (cross-linked enzyme aggregate), laccase formed by multiple linking through a cross linker showed more enhanced stability and performance during starch oxidation, and CLEA of another enzyme, nitrile hydratase showed an excellent increase in activity in the conversion of acrylonitrile to acrylamide, and did not lose activity during 36 recycles. In addition, many proteins form complexes in cells to perform complex functions, which are known to be due to the proximity effect of proteins. For example, the cellulase (Novozymes Cellic® CTec3), which is produced by preparing enzymes necessary for lignocellulose degradation, such as, cellulase, beta glucosidase (β-glucosidase), hemicellulase and the like in the form of a complex mixture using a scaffold, is known to exhibit a 3.5-fold or more increased effect in the degradation of lignocellulose. In addition, such a protein in the multimeric form exhibits a channeling effect. That is, if enzymes involved in a coupled reaction are present adjacent to each other, the transfer of the intermediate is efficient and the efficiency of the entire reaction is greatly increased. In addition, it is proposed to be desirable for an increase in its efficiency to use a homogeneous or heterogeneous protein in a multimeric form when analyzing an any substance using a protein immobilized on a bead or a substrate, or separating and/or purifying a substance to be detected. As described above, although the protein in the multimeric form provides various advantages in industrial and medical applications, it has been known that it is difficult to fabricate a protein having such a structure. For example, there is a method of developing and producing a multimeric protein as a new fusion enzyme by designing in-frame at the genetic stage. However, since a new protein must be designed and produced, it takes a long time to develop it and it is difficult to fuse two or more enzymes in reality. In addition, in the case of a method of fabricating a protein multimer construct (CLEA) using a chemical cross linker, the activity may be inhibited because a chemical bond does not occur at a specific site but can occur anywhere on the protein surface. Proteins that form a multimer construct must be capable of being prepared through synthesis or microbial expression, and the active sites of these proteins must not be disturbed.

A method of using ubiquitin has been proposed as a method for separating and/or purifying a protein of interest. It is the method in which first, a gene encoding a protein bound to ubiquitin is expressed in prokaryotic cells to prepare a fusion protein linked to ubiquitin, and then treated with ubiquitin cleavage enzyme to effectively separate and purify only the protein of interest from the ubiquitin fusion protein. U.S. Patent Application No. 10/504,785 relates to the expression of a recombinant gene and the purification of the expressed protein, and it describes that the fusion protein is prepared in which the nucleotide encoding the C-terminal domain of the ubiquitin-like protein (Ubl) is operatively bound to the nucleotide encoding the protein of interest, and it is expressed in a host cell. Korean Patent Application No. 10-2005-0050824 describes the use of ubiquitin as a fusion partner in expressing a recombinant protein. In addition, Korean Patent Application No. 10-2015-0120852 relates to the use of an ubiquitin column for purifying a protein, and describes that a polyubiquitin chain is loaded on the column, and the protein is purified using in vitro ubiquitination including E2. In addition, U.S. Patent Application No. 12/249,334 is to solve the problem of water solubility and folding, which is a problem in preparing by expressing a recombinant protein, and describes the use of SUMO having a cleavage site recognized by Ulp1 protease (Ubl-specific protease 1) for facilitating expression, separation and purification of the recombinant protein, and for increasing the activity of the protein. However, these methods only describe the use of ubiquitin for protein expression, and do not describe or suggest the production of a protein in a multimeric form, and since the protein to be separated and purified randomly binds to ubiquitin, these methods still have a limit to separation or analysis efficiency.

Accordingly, the present inventors have made ceaseless efforts to develop a method for preparing a protein in a multimeric form having a high degree of integration without inhibiting the activity of the protein. As a result, a biomolecule bound to ubiquitin was recombinantly expressed from a host cell and was reacted in vitro with an enzyme related to ubiquitination to form a linear polyfunctional multimeric biomolecule polymer bound to a polyubiquitin scaffold. Based on the above, the present inventors completed the present invention.

### Detailed Description of the Invention

### Technical Problem

As described above, an object of the present invention is to provide a method of immobilizing or cross-linking biomolecules such as proteins in vitro using a Ubiquitin C-terminal Tag (UCT).

Another object of the present invention is to provide a linear polyfunctional multimeric biomolecule in which a target biomolecule is bound to a polyubiquitin scaffold and a method for preparing the same.

Another object of the present invention is to provide a construct in which the linear polyfunctional multimeric biomolecule is immobilized and a method for preparing the same.

Another object of the present invention is to provide a method for separating and purifying a target material using the linear polyfunctional multimeric biomolecule.

Another object of the present invention is to provide a method of analyzing, or separating and purifying a target material that binds to the biomolecule using the linear polyfunctional multimeric biomolecule.

Another object of the present invention is to provide a method for site-specifically binding two or more biomolecules and/or small molecule chemical compounds using polyubiquitin as a linker.

Another object of the present invention is to provide the use of polyubiquitin for site-specifical binding of two or more biomolecules and/or small molecule chemical compounds.

Another object of the present invention is to provide a pharmaceutical composition comprising the linear polyfunctional multimeric biomolecule of the present invention.

### Solution to Problem

In order to achieve the above objects, the present invention provides a method for preparing a linear polyfunctional multimeric biomolecule, wherein the method comprises (i) recombinantly expressing a biomolecule to which a ubiquitin C-terminal tag is fused or bound by a linker from a host cell including a prokaryotic cell or a eukaryotic cell, and (ii) adding E1, E2 and E3 enzymes for ubiquitination, or E1 and E2 enzymes for ubiquitination to a cell lysate of the host cell and reacting them, wherein the biomolecule is bound to a polyubiquitin scaffold formed of two or more covalently bonded ubiquitins, and the biomolecule comprises two or more binding moieties, each specific for different binding sites. Accordingly, in the present invention, an initiator that initiates the formation of a linear polyfunctional multimeric biomolecule polymer or complex may be E3, E2, E1, a free ubiquitin, or a target substrate of E3. Here, the E2 enzyme may bind to the lysine at the 48th or 63rd amino acid residue of ubiquitin, and the E2 enzyme may be an E2-25K ubiquitin conjugating enzyme, or a ubiquitin conjugating enzyme complex Ucb13-MMS2.

In one embodiment of the present invention related thereto, the recombinantly expressed biomolecule is one in which a C-terminal portion of the 76th amino acid residue, glycine, of a ubiquitin C-terminal tag is extended by 1 to 50 amino acids, and the method may further comprise adding DUB (deubiquitinating enzyme), for example, UH1, YUH2, UCH-L1, UCH-L2 or UCH-L3, to the recombinantly expressed biomolecule before or after the reaction of the above step (ii).

In one embodiment of the present invention related thereto, the biomolecule has active sites that specifically bind to other biomolecules, small molecule chemical compounds or nanoparticles or the like, and the biomolecule may be one or more selected from the group consisting of an enzyme, a protein, a peptide, a polypeptide, an antibody, DNA and RNA, but is not limited thereto, and ATP may be further added to the above step (ii) and reacted with them. Advantageously, each of the enzyme, protein, peptide, polypeptide, antibody, DNA and RNA may be homogeneous or heterogeneous. That is, the monomers constituting the linear polyfunctional multimer complex of the present invention may be homogeneous or heterogeneous proteins, or proteins and peptides or antibodies, respectively, and the monomers of various types of biomolecules may be formed into a linear polyfunctional multimer complex as necessary. Here, the biomolecule may be one or more selected from the group consisting of protein A, protein G, lysin, endolysin, protease, hydrolase, oxidoreductase, lyase, affinity ligand and receptor, but is not limited thereto. In addition, the biomolecule may be one or more selected from the group consisting of insulin, insulin analogue, glucagon, glucagon-like peptides (GLP-1 and the like), GLP-1/glucagon dual agonist, exendin-4, exendin-4 analogue, insulin secreting peptide and an analogue thereof, human growth hormone, growth hormone releasing hormone (GHRH), growth hormone releasing peptide, granulocyte colony stimulating factor (G-CSF), anti-obesity peptide, G-protein-coupled receptor, leptin, GIP (gastric inhibitory polypeptide), interleukins, interleukin receptors, interleukin binding proteins, interferons, interferon receptors, cytokine binding proteins, macrophage activator, macrophage peptide, B cell factor, T cell factor, suppressive factor of allergy, cell necrosis glycoprotein, immunotoxin, lymphotoxin, tumor necrosis factor (TNF), tumor inhibitory factor, metastasis growth factor, alpha-1 antitrypsin, albumin, α-lactalbumin, apolipoprotein-E, erythropoietin (EPO), high glycosylated erythropoietin, angiopoietins, hemoglobin, thrombin, thrombin receptor activating peptide, thrombomodulin, blood factors VII, VIIa, VIII, IX, and XIII, plasminogen activator, fibrin-binding peptide, urokinase, streptokinase, hirudin, protein C, C-reactive protein, renin inhibitor, collagenase inhibitor, superoxide dismutase, platelet derived growth factor, epithelial growth factor, epidermal growth factor, angiostatin, angiotensin, bone formation growth factor, bone formation promoting protein, calcitonin, atriopeptin, cartilage inducing factor, elcatonin, connective tissue activator, tissue factor pathway inhibitor, follicle stimulating hormone (FSH), luteinizing hormone (LH), luteinizing hormone releasing hormone (LHRH), nerve growth factors, parathyroid hormone (PTH), relaxin, secretin, somatomedin, adrenal cortical hormone, cholecystokinin, pancreatic polypeptide, gastrin releasing peptide, corticotropin releasing factor, thyroid stimulating hormone (TSH), autotaxin, lactoferrin, myostatin, receptor, receptor antagonist, fibroblast growth factor, adiponectin, interleukin receptor antagonist, aspartate, 6xHis, chitin binding domain, GST, thrombin, FLAG tag, cell surface antigen, virus derived vaccine antigen, monoclonal antibody, polyclonal antibody and antibody fragments, but is not limited thereto.

In one embodiment of the present invention related thereto, E1, E2 and E3 used for in vitro ubiquitination may be selected and used in any combination. For example, if the E2 is UBCH5A (UBE2D1), it may be selected from the group consisting of RSP5 (UniProt ID. P39940), DTX2 (UniProt ID. Q86UW9), DTX3 (UniProt ID. Q8N9I9), MID1 (UniProt ID. 015344), RING1 (UniProt ID. Q06587), RNF11 (UniProt ID. Q9Y3C5), RNF111 (UniProt ID. Q6ZNA4), RNF126 (UniProt ID. Q9BV68), RNF115 (UniProt ID. Q9Y4L5), RNF14 (UniProt ID. Q9UBS8), RNF185 (UniProt ID. Q96GF1), RNF2 (UniProt ID. Q99496), RNF5 (UniProt ID. Q99942), TRAF6 (UniProt ID. Q9Y4K3), TRIM8 (UniProt ID. Q9BZR9), ZNRF1 (UniProt ID. Q8ND25), XIAP (UniProt ID. P98170), and TRIM39 (UniProt ID. Q9HCM9). In addition, if the E2 is UBC7, the E3 may be DOA10 (UniProt ID. P40318), UFD4 (UniProt ID. P33202), HRD1 (UniProt ID. Q08109) or HRD3 (UniProt ID. Q05787), and if the E2 is UBE2W (UBC16), as the E3 interacting with it, MARCH5 (UniProt ID. Q9NX47) or RNF5 (UniProt ID. Q99942) may be used, but is not limited thereto.

In another embodiment of the present invention related thereto, advantageously, the ubiquitin C-terminal tag is one in which the lysine at the 48th or the 63rd amino acid residue from the N-terminus of the ubiquitin is substituted with alanine, or more advantageously, all lysines except for one lysine at any position are deleted or substituted with amino acids other than lysine. In addition, all lysines of the ubiquitin except for the lysine at the 11th, 48th, or 63rd amino acid residue starting from the N-terminal Met1 of the ubiquitin may be substituted with arginine. In addition, the ubiquitin C-terminal tag may be one in which two or more ubiquitins may be repeatedly linked in a head-to-tail form. In this case, the ubiquitin linked in the head-to-tail form may be one in which the glycines at the 75th and 76th amino acid residue from the N-terminus may be substituted with other amino acids including valine, or the leucine at the 73^{rd} amino acid residue may be substituted with proline.

In another embodiment of the present invention related thereto, the reaction of the above step (ii) is carried out in the presence of a substrate or substrates or a bead for immobilization of ubiquitin, and E3 ligase may be attached to one terminus of the biomolecule.

In addition, the present invention provides a linear multimeric biomolecule polymer comprised of a polyubiquitin scaffold and a biomolecule, wherein the polyubiquitin scaffold is formed by linking two or more ubiquitins, for example, through covalent bonds, and the biomolecule is each bound to the ubiquitin. The biomolecule is preferably each bound to the N-terminus of the ubiquitin. The initiator that initiates the formation of a linear multimeric biomolecule polymer may be E3, E2, E1, a free ubiquitin, or a substrate. In addition, the linear multimeric biomolecule polymer may be comprised of 2 to 20 biomolecules.

In one embodiment of the present invention related thereto, the biomolecule may be one or more selected from the group consisting of an enzyme, a protein, a peptide, a polypeptide, an antibody, DNA and RNA, miRNA, siRNA, and a small molecule chemical compound. Advantageously, each of the protein, peptide, polypeptide, antibody, DNA and RNA may be of different types.

In another embodiment of the present invention related thereto, the linear polyfunctional multimeric biomolecule complex or polymer may be originated from E3, E2, E1, a free ubiquitin, or a substrate, and the E3 may be Rsp5, WWP1, nedd4 or XIAP, or a minimal catalytic domain thereof, and the E2 may be Ubc7, Ubch5a, E2-25K (GenBank ID-U58522.1), Ubc13-MMS2 (Unipot ID-P52490) complex, or a minimal catalytic domain thereof.

In another embodiment of the present invention related thereto, the ubiquitin is one in which the lysine at the 48th or the 63rd amino acid residue from the N-terminus of the ubiquitin is substituted with alanine, or more advantageously, other lysines except for one lysine at any position thereof are deleted or substituted with amino acids other than lysine. In addition, the free ubiquitin may be one in which other lysines except for one lysine at any position thereof are deleted or substituted with amino acids other than lysine, and it may be one in which all lysines except for the lysine at the 11th, 48th, or 63rd amino acid residue from the N-terminus are substituted with arginine. In addition, the free ubiquitin may be one in which a C-terminal portion of the glycine at the 76th amino acid residue from the N-terminus thereof may be extended by 1 to 50 amino acids, and the amino acid may be aspartate, 6xHis tag, or GST tag, and the protein may have PPPY for Rsp5 or Nedd4-1,2. The biomolecule may be linked to the N-terminal Met1 of the free ubiquitin, and an initiator, for example, E3, E2, E1, a free ubiquitin or a substrate may be attached to one terminus of the biomolecule. In addition, the substrate may be a protein that comprises an amino acid sequence that recognizes E3 ligase and comprises one or more lysine to which ubiquitin is capable of binding.

In another embodiment of the present invention related thereto, the present invention also provides a method of separating a biomolecule expressed in a host cell in vitro, wherein the method comprises (i) recombinantly expressing a biomolecule to which a ubiquitin C-terminal tag is fused or bound by a linker from a host cell including a prokaryotic cell, a eukaryotic cell, or an animal cell; (ii) adding E1, E2 and E3 proteins for ubiquitination to a cell lysate of the host cell and reacting them to form a linear polyfunctional multimeric biomolecule complex in which a biomolecule to be separated and purified is bound to a polyubiquitin scaffold formed of two or more covalently bonded ubiquitins; and (ii) separating the linear multimeric biomolecule complex.

In one embodiment of the present invention related thereto, the biomolecule is one or more selected from the group consisting of a protein, a peptide, a polypeptide, an antibody, DNA and RNA, and ATP is further added to the above step (ii) and reacted. Advantageously, each of the protein, peptide, polypeptide, antibody, DNA and RNA may be of different types.

In another embodiment of the present invention related thereto, advantageously, the ubiquitin C-terminal tag is one in which the lysine at the 48th or the 63rd amino acid residue is substituted with alanine, or more advantageously, all lysines except for one lysine at any position are deleted or substituted with amino acids other than lysine.

In another embodiment of the present invention related thereto, the reaction of the above step (ii) is carried out in the presence of a substrate or substrates or a bead for immobilization of ubiquitin, and E3 ligase may be attached to one terminus of the biomolecule.

The present invention provides a separation column comprising a linear polyfunctional multimeric biomolecule complex. In one embodiment related thereto, the biomolecule may be a protein or an antibody. In the present invention, the linker may be a peptide consisting of 3 to 50 amino acids, but is not limited thereto.

In another embodiment of the present invention, the present invention provides a linear polyfunctional multimeric biomolecule polymer comprised of a polyubiquitin scaffold and a biomolecule, wherein the linear polyfunctional multimeric biomolecule polymer comprises two or more binding moieties that are specific for different binding sites, and the polyubiquitin scaffold is formed of two or more covalently bonded ubiquitins; the biomolecule has active sites that specifically bind to other biomolecules, small molecule chemical compounds or nanoparticles or the like, and the biomolecule is bound to the N-terminus, the C-terminus, or both the N-terminus and the C-terminus of the ubiquitin. Here, the biomolecule polymer may be comprised of 2 to 4 biomolecules, and the biomolecule may be bound by a linker to the N-terminus, the C-terminus, or both the N-terminus and the C-terminus of the ubiquitin. the linker may be a combination of 1 to 6 repeats of GGGGS or EAAAK. In this case, the biomolecule bound to the N-terminus of the ubiquitin is the distal end of the linear multimeric biomolecule polymer, and the biomolecule bound to the C-terminus or the N-terminus or both the C-terminus and the N-terminus of the ubiquitin is the proximal end of the linear multimeric biomolecule polymer. In other embodiment related thereto, the polyubiquitin scaffold may be formed by covalently linking a donor ubiquitin in which all lysines of the ubiquitin are substituted with arginine, and an acceptor ubiquitin in which all lysines of the ubiquitin except for the lysine at the 11th, 48th, or 63rd amino acid residue are substituted with arginine, and the leucine at the 73rd amino acid residue from the N-terminus of the ubiquitin may be substituted with proline.

In other embodiment related thereto, the linear polyfunctional multimeric biomolecule polymer may be comprised of 2 to 20 biomolecules.

In other embodiment related thereto, the biomolecule may be one or more selected from the group consisting of an enzyme, a protein, a peptide, a polypeptide, an antibody, an antibody fragment, DNA and RNA, and the biomolecule may be one or more selected from the group consisting of protein A, protein G, lysin, endolysin, protease, hydrolase, oxidoreductase, lyase, affinity ligand and receptor. In addition, the biomolecule may be selected from the group consisting of insulin, insulin analogue, glucagon, glucagon-like peptides (GLP-1 and the like), GLP-1/glucagon dual agonist, exendin-4, exendin-4 analogue, insulin secreting peptide and an analogue thereof, human growth hormone, growth hormone releasing hormone (GHRH), growth hormone releasing peptide, granulocyte colony stimulating factor (G-CSF), anti-obesity peptide, G-protein-coupled receptor, leptin, GIP (gastric inhibitory polypeptide), interleukins, interleukin receptors, interleukin binding proteins, interferons, interferon receptors, cytokine binding proteins, macrophage activator, macrophage peptide, B cell factor, T cell factor, suppressive factor of allergy, cell necrosis glycoprotein, immunotoxin, lymphotoxin, tumor necrosis factor (TNF), tumor inhibitory factor, metastasis growth factor, alpha-1 antitrypsin, albumin, α-lactalbumin, apolipoprotein-E, erythropoietin (EPO), high glycosylated erythropoietin, angiopoietins, hemoglobin, thrombin, thrombin receptor activating peptide, thrombomodulin, blood factors VII, VIIa, VIII, IX, and XIII, plasminogen activator, fibrin-binding peptide, urokinase, streptokinase, hirudin, protein C, C-reactive protein, renin inhibitor, collagenase inhibitor, superoxide dismutase, platelet derived growth factor, epithelial growth factor, epidermal growth factor, angiostatin, angiotensin, bone formation growth factor, bone formation promoting protein, calcitonin, atriopeptin, cartilage inducing factor, elcatonin, connective tissue activator, tissue factor pathway inhibitor, follicle stimulating hormone (FSH), luteinizing hormone (LH), luteinizing hormone releasing hormone (LHRH), nerve growth factors, parathyroid hormone (PTH), relaxin, secretin, somatomedin, adrenal cortical hormone, cholecystokinin, pancreatic polypeptide, gastrin releasing peptide, corticotropin releasing factor, thyroid stimulating hormone (TSH), autotaxin, lactoferrin, myostatin, receptor, receptor antagonist, fibroblast growth factor, adiponectin, interleukin receptor antagonist, cell surface antigen, virus derived vaccine antigen, monoclonal antibody, polyclonal antibody and antibody fragments.

In other embodiment related thereto, the linear polyfunctional multimeric biomolecule polymer may be originated from E3, E2, E1, a free ubiquitin, or a substrate, and the E3 may be Rsp5, WWP1, nedd4 or XIAP, or a minimal catalytic domain thereof, and the E2 may be Ubc7, Ubch5a, E2-25K, Ubc13-MMS2 complex, or a minimal catalytic domain thereof. Here, the free ubiquitin may be one in which other lysines except for one lysine at any position thereof are deleted or substituted with amino acids other than lysine. In addition, the free ubiquitin may be one in which all lysines except for the lysine at 11th, 48th, or 63rd aminio acid residue from the N-terminus thereof are substituted with arginine, and the free ubiquitin may be one in which a C-terminal portion of the glycine at the 76th amino acid residue from the N-terminus thereof is extended by 1 to 50 amino acids, or the free ubiquitin may be extended by aspartate, 6xHis tag, or GST tag, or the biomolecule may be linked to the N-terminal Met1 of the free ubiquitin. In other embodiment related thereto, E3, E2, E1, a free ubiquitin or a substrate may be attached to one terminus of the biomolecule as an initiator, and the substrate may be a protein that comprises an amino acid sequence that recognizes E3 ligase and comprises one or more lysine to which ubiquitin is capable of binding, and the protein may comprise PPPY for Rsp5 or Nedd4-1,2. In other embodiment related thereto, the ubiquitin may be one in which other lysines except for one lysine at any position thereof are deleted or substituted with amino acids other than lysine.

In another aspect of the present invention, herein is provided a pharmaceutical composition, wherein the composition comprises the linear polyfunctional multimeric biomolecule of the present invention and a pharmaceutically acceptable carrier, and has an increased in vivo stability. The pharmaceutical composition according to the present invention may be administered through various routes, for example, orally, transdermally, subcutaneously, intravenously or intramuscularly into the body. In addition, the pharmaceutical composition according to the present invention may be formulated using a method well known in the art. The formulation may be in the form of a tablet, pill, powder, sachet, elixir, suspension, emulsion, solution, syrup, aerosol, soft or hard gelatin capsule, sterile injectable solution, sterile powder, and the like. Examples of suitable carriers, excipients and diluents for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, and the like, which may be used. The formulation may further comprise fillers, anticoagulants, lubricants, wetting agents, flavoring agents, emulsifying agents, preservatives, and the like. The actual dosage of the biomolecule of the present invention is determined depending on the type of the physiologically active polypeptide as an active ingredient, along with various related factors such as the disease to be treated, the route of administration, the age, sex and weight of the patient, and the severity of the disease.

The term "polyfunctional" used in the present invention is used interchangeably with "multifunctional" or "multivalent," and is understood to have the same meaning in the present invention.

The term "polyfunctional multimeric biomolecule" used in the present invention is defined as a biomolecule comprising two or more binding moieties, each specific for different binding sites.

The terms "complex," "polymer," "conjugate," and "construct" used in the present invention are used interchangeably in the present specification as having the same meaning when these terms refer to the linear polyfunctional multimeric biomolecule of the present invention bound to polyubiquitin by a linker.

The term "ubiquitin C-terminal tag" used in the present invention may be understood as a ubiquitin forming the initiating portion in the formation of a polyubiquitin.

### Effect of the Invention

According to the present invention, since the linkage between linear polyfunctional multimeric biomolecule polymers or complexes is made by UCT, polyubiquitin formed by linkage of UCT may act as a rigid scaffold or linker that maintains the spacing and orientation between biomolecules bound to UCT. In addition, since the enzymatic reaction (E1-E2-E3) is used for the binding between UCTs, the biomolecule-UCT expressed in a host cell can be easily used in the form of a cell lysate mixture without separate process steps of separation and purification. In addition, the biomolecule of the present invention may be one or more selected from the group consisting of a protein, a peptide, a polypeptide, an antibody, an antibody fragment, DNA and RNA, and for example, a heterogeneous protein may be used to give the modularized functionality to a linear polyfunctional multimer polymer. Enzymes made from linear polymers by enzyme proximity effects exhibit a more enhanced effect in catalytic functionality and stability. When heterogeneous enzymes constituting a coupled reaction are made into a linear multimer construct, a synergistic effect is achieved in that the overall reaction efficiency is higher than that of a bulk mixture due to a channeling effect. In addition, since the target biomolecule-UCT can be attached to the stationary phase using an enzymatic reaction, the biomolecule-UCT does not need to be purified and separated purely. Therefore, a linear multimer construct is synthesized and immobilized in a crude-mixture comprising a biomolecule-UCT, such as cell lysates or culture media. Therefore, the immobilized enzyme can be produced economically.

### Brief Description of Drawings

Figure 1 schematically shows the process of preparing the linear polyfunctional multimer fusion protein (Ubstac) of the present invention.
Figures 2 and 3 show results of confirming the UCT fusion protein in a multimeric form formed by the Ubstac reaction of the present invention.
Figure 4 schematically shows the preparation of the linear polyfunctional multimer fusion protein of the present invention and the use thereof by immobilization.
Figure 5 shows the results of Ubstac preparation using only E1-E2.

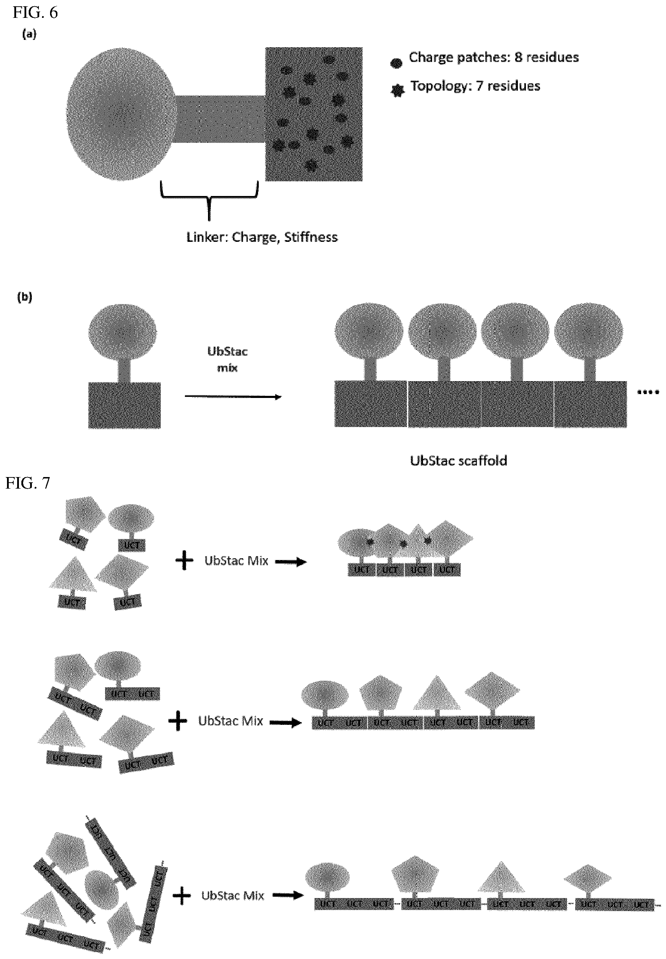

Figure 6 schematically shows the preparation of the linear polyfunctional multimer fusion protein of the present invention and the use thereof by immobilization.
Figure 7 schematically shows the head-to-tail UCT and UbStac method.
Figure 8 is a result of confirming by SDS-PAGE after purification of xylose reductase (XR) prepared according to the present invention by GPC.
Figure 9 is a result of confirming by SDS-PAGE after purification of oxaloacetate decarboxylase (OAC) prepared according to the present invention by GPC.
Figure 10 is a result of confirming by SDS-PAGE after purification of xylitol dehydrogenase (XDH) prepared according to the present invention by GPC.
Figure 11 is a result of confirming by SDS-PAGE after purification of triose-phosphate isomerase (TIM) prepared according to the present invention by GPC.
Figure 12 is a result of confirming by SDS-PAGE after purification of aldolase (ALD) prepared according to the present invention by GPC.
Figure 13 is a result of confirming by SDS-PAGE after purification of fructose 1,6-bisphosphatase (FBP) prepared according to the present invention by GPC.
Figure 14 is a result of confirming by SDS-PAGE after purification of pyruvate oxidase (POPG) prepared according to the present invention by GPC.
Figure 15 is a result of analysis of the activity of xylose reductase.
Figure 16 is a result of analysis of the stability of xylose reductase.
Figure 17 is a result of analysis of the activity of oxaloacetate decarboxylase.
Figure 18 is a result of analysis of the stability of oxaloacetate decarboxylase.
Figure 19 is a result of analysis of the activity of xylitol dehydrogenase.
Figure 20 is a result of analysis of the stability of xylitol dehydrogenase.
Figure 21 is a result of analysis of the activity of pyruvate oxidase.
Figure 22 shows a result of the Ubstac of the structure to which three enzymes, TIM, ALD and FBP are bound.
Figure 23 shows the synergistic effect by TIM, ALD and FBP enzymes.
Figure 24 is a result of preparing and confirming Protein A and Protein G linear polyfunctional multimer complexes.
Figure 25 is a result of preparing and confirming hGH in which aspartate is extended at the C-terminal portion of the glycine at the 76th amino acid residue of the ubiquitin C-terminal tag.
Figure 26 is a result of preparing and confirming the polymer originated from E3.
Figure 27 is a result of preparing and confirming the polymer of hGH expressed in an extended form with aspartate at the C-terminus of a UCT repeatedly linked in a head-to-tail form.
Figure 28 shows that the binding activity of human derived IgG to the beads on which the Protein A polymer is immobilized is increased compared to the beads on which the Protein A monomer is immobilized.
Figure 29 schematically shows the structure of a linear polyfunctional multimeric biomolecule polymer bound to the N-terminus, the C-terminus, or both the N-terminus and the C-terminus of the ubiquitin, respectively.

### Best Mode for Carrying out the Invention

In an embodiment, the present invention provides a method for preparing a linear polyfunctional multimeric biomolecule, wherein the method comprises (i) recombinantly expressing a biomolecule to which a ubiquitin C-terminal tag is fused or bound by a linker from a host cell including a prokaryotic cell or a eukaryotic cell, and (ii) adding E1, E2 and E3 enzymes for ubiquitination, or E1 and E2 enzymes for ubiquitination to a cell lysate of the host cell and reacting them, wherein the biomolecule is bound to a polyubiquitin scaffold formed of two or more covalently bonded ubiquitins, and the biomolecule comprises two or more binding moieties, each specific for different binding sites. Accordingly, in the present invention, an initiator that initiates the formation of a linear polyfunctional multimeric biomolecule polymer or complex may be E3, E2, E1, a free ubiquitin, or a target substrate of E3. Here, the E2 enzyme may bind to the lysine at the 48th or 63rd amino acid residue of ubiquitin, and the E2 enzyme may be an E2-25K ubiquitin conjugating enzyme, or a ubiquitin conjugating enzyme complex Ucb13-MMS2.

In another embodiment, the present invention provides a linear polyfunctional multimeric biomolecule polymer comprised of a polyubiquitin scaffold and a biomolecule, wherein the polyubiquitin scaffold is formed by linking two or more ubiquitins, for example, through covalent bonds, and the biomolecule is each bound to the ubiquitin. The biomolecule is preferably each bound to the N-terminus of the ubiquitin. The initiator that initiates the formation of a linear multimeric biomolecule polymer may be E3, E2, E1, a free ubiquitin, or a substrate. In addition, the linear multimeric biomolecule polymer may be comprised of 2 to 20 biomolecules.

Hereinafter, the present invention is to be described in more detail through the following examples. These examples are only for describing the present invention in more detail, and it will be apparent to those of ordinary skill in the art that the scope of the present invention is not limited by these examples according to the gist of the present invention.

### Mode for Carrying out the Invention

### Preparation Example

### Preparation Example 1: Cloning, expression and purification of C-terminal fusion protein

The gene encoding the UCT (Ubiquitin C-terminal Tag) (SEQ ID NO: 1) protein fusion used in the examples of the present invention was produced on request by Genscript Inc.

In order to prepare a Ub out gene construct that does not comprise a ubiquitin tag at the C-terminus, fast cloning system (Li C, Wen A, Shen B, Lu J, Huang Y, Chang Y (2011). Fast cloning: a highly simplified, purification-free, sequence- and ligation-independent PCR cloning method. BMC Biotechnol 11, 92.) was used. This method is a technology capable of linking genes (insertion, removal or substitution) in which if the PCR product is directly treated with only Dpn1 in the absence of a restriction enzyme and ligase, Dpn1 plays a role of a restriction enzyme and ligase through a mechanism that has not yet been identified along with polymerase. In this method, using a primer designed to overlap both terminus with Phusion polymerase (Thermo Fisher Scientific), PCR (95 °C for 3 minutes, 95 °C for 15 seconds - 55 °C for 1 minute - 72 °C for 1 minute/kb 18 times repeated, 72 °C for 5 minutes, 12 °C for 20 minutes) was carried out on all vectors except for the region to be deleted. Next, the resulting PCR product was subjected to Dpn1 treatment for 1 hour at 37 °C, and transformed into *E. coli* DH5α (Novagen), and then the plasmid of interest was obtained. All gene constructs were identified by commercial DNA sequencing.

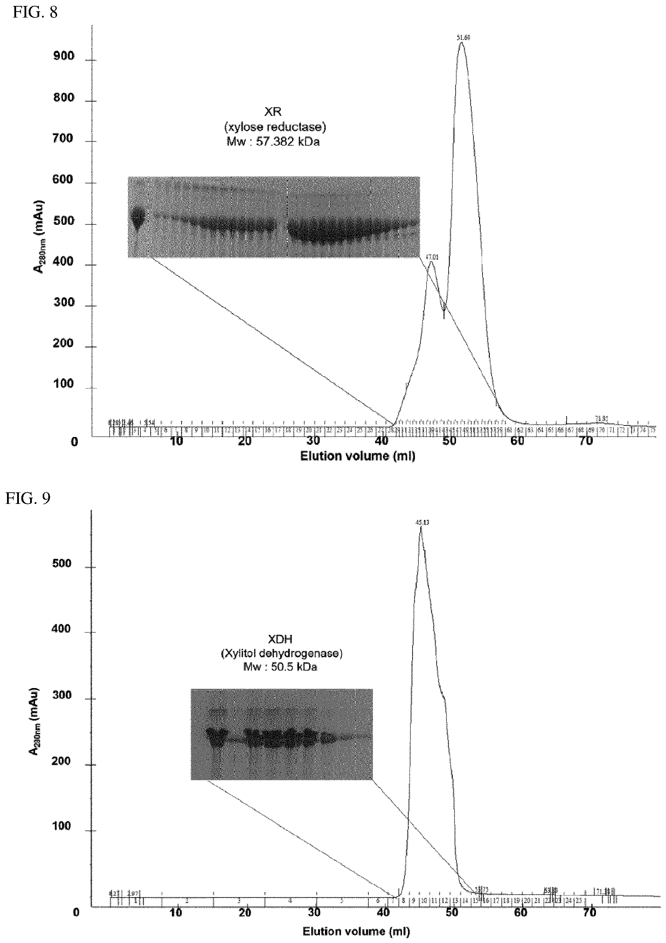

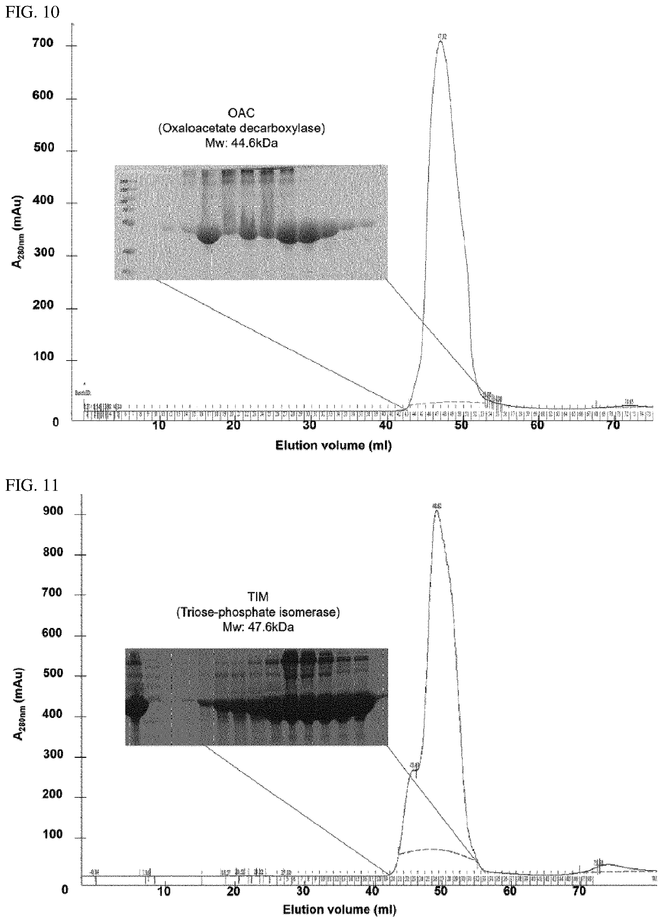

For overexpression of UCT fusion protein, each gene construct was transformed into *E*. *coli* BL21 DE3 (Novagen) (XR, TIM, ALD), *Rosetta* pLysS DE3 (Novagen) (XDH, OAC, POPG), *Origami2* DE3 (Novagen) (FBP) strains. Cells comprising the protein expression plasmid (pET21a, Genscript) were incubated in LB medium (Miller) at 37 °C. When the OD₆₀₀ value reached about 0.6, the protein expression was induced with 250 µM of isopropyl β-D-1-thiogalactopyranoside (isopropyl-beta-D-thiogalactopyranoside) (IPTG) at 16 °C for 20 hours. Next, after centrifugation (at 3,500 rpm at 4 °C for 15 minutes), cell pellet was resuspended in lysis buffer (20 mM Tris-HCl pH 8.0, 500 mM NaCl₂, 20 mM imidazole), and lysed by sonication (50% amplitude, pluse on 3 seconds-off 5 seconds, final 15 minutes). Then, the lysate was further centrifuged at 14,000 rpm at 4 °C for 30 minutes. The water soluble fraction of the protein comprising the N-terminal His-tag was purified by gel filtration chromatography using Superdex 75 pg gel filtration column 16/600 (GE Healthcare) pre-equilibrated with nickel affinity and FPLC buffer (Ni-NTA Agarose, QIAGEN, 20 mM Tris-HCl pH 8.0, 150 mM NaCl₂). All UCT proteins were concentrated to 100 µM for analysis of the enzyme activity. All target proteins were evaluated by SDS-PAGE. Figures 8 to 14 show the results of confirming the target proteins. The UCT fusion proteins used in the present invention are shown in Table 1 below.

**[Table 1]**

| UCT protein fusion | Molecular weight (kDa) | SEQ ID NO |
|---|---|---|
| Xylose reductase (XR) | 57.382 | SEQ ID NO: 2 |
| Xylitol dehydrogenase (XDH) | 59.1 | SEQ ID NO: 3 |
| Oxaloacetate decarboxylase (OAC) | 44.6 | SEQ ID NO: 4 |
| Triose-phosphate isomerase (TIM) | 47.6 | SEQ ID NO: 5 |
| Aldolase (ALD) | 55.563 | SEQ ID NO: 6 |
| Fructose 1,6-bisphosphatase (FBP) | 49.3 | SEQ ID NO: 7 |
| Pyruvate oxidase (POPG) | 86.032 | SEQ ID NO: 8 |

### Preparation Example 2: Preparation of Ubstac linear construct

In the present invention, the reaction for preparing a linear polyfunctional multimeric fusion protein was designated as Ubstac reaction, respectively. The Ubstac reaction (a total volume of 50 µL) was carried out in the Ubstac buffer (25 mM HEPES (Sigma-aldrich), pH 7.5, 50 mM NaCl, 4 mM MgCl₂), and the Ubstac mixture for the Ubstac reaction (0.5 µM E1, 5 µM E2, 1 µM E3, 4 mM ATP) was added to the UCT protein fusion of the present invention to initiate the reaction. The ratio of protein used in the reaction was at a concentration of 10 uM to 20 uM UCT protein fusion per 1 uM E3 enzyme (a ratio of 1:10 to 1:20), which was a condition set for the purpose so that at least 10 fusion monomers form a linear polyfunctional multimer within 1 hour through the Ubstac reaction. The E1, E2 and E3 used in the present invention are as follows, respectively:

**[Table 2]**

| Category | Name | SEQ ID NO |
|---|---|---|
| E1 | Yeast UBE 1 | SEQ ID NO: 9 |
| | Ubch5a [*Homo sapiens*] (UniProtKB - P51668) | SEQ ID NO: 10 |
| | Ubch7 [*Homo sapiens*] (UniProtKB - P68036) | SEQ ID NO: 11 |
| E2 | E2-25K [*Homo sapiens*] (UniProtKB - P61086) | SEQ ID NO: 12 |
| | Ubc13 [*Saccharomyces cerevisiae*] (UniProtKB - P52490) | SEQ ID NO: 13 |
| | MMS2 (UEV - Ubiquitin-conjugating enzyme variant) | SEQ ID NO: 14 |
| | RSP5 (UniProt ID. P39940) | SEQ ID NO: 15 |
| E3 | DOA10 (UniProt ID. P40318) | SEQ ID NO: 16 |
| | MARCH5 (UniProt ID. Q9NX47) | SEQ ID NO: 17 |

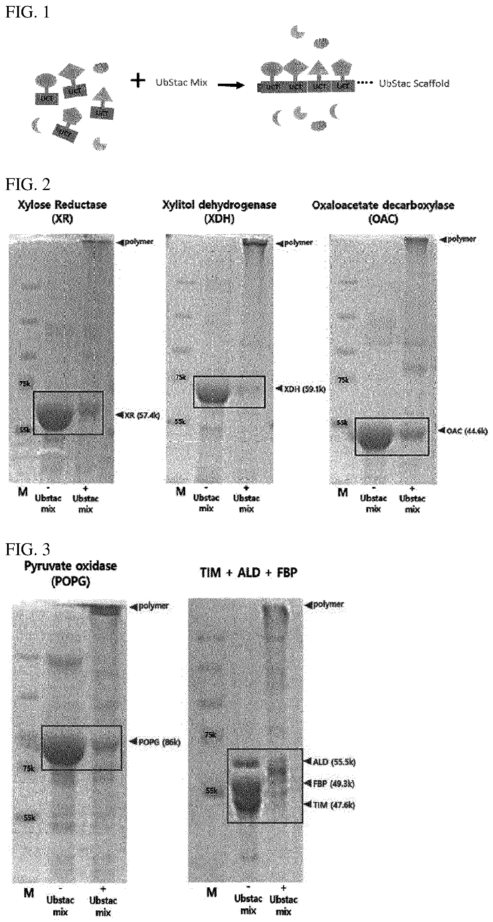

The Ubstac reaction was carried out by shaking at room temperature for 1 hour. Figure 1 schematically shows the Ubstac reaction of the present invention, and Figures 2 and 3 show results of confirming the UCT fusion protein in a multimeric form formed by the Ubstac reaction.

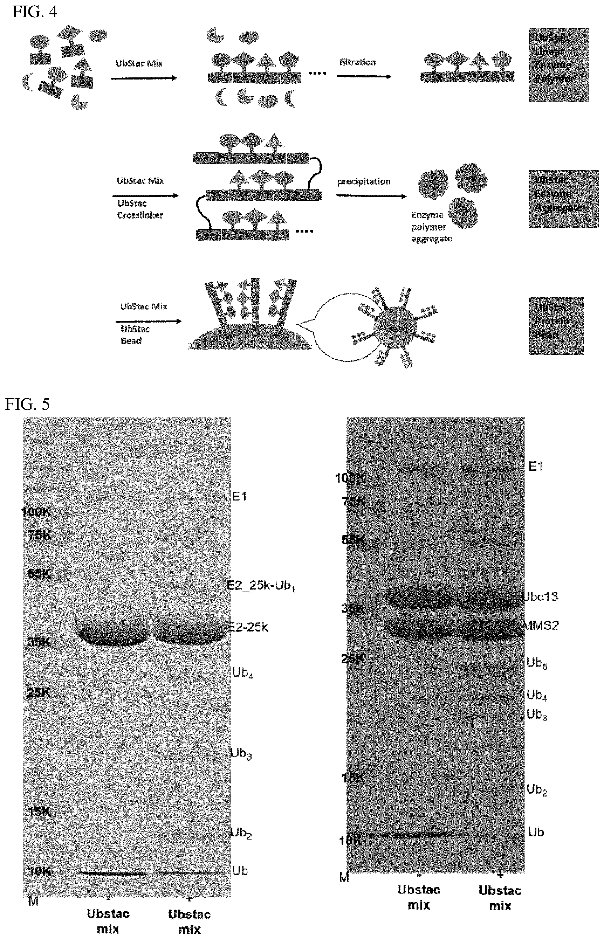

In Figure 4, the first drawing schematically shows a process of preparing a Ubstac linear enzyme polymer by reacting a Ubstac mixture with a ubiquitin C terminal tagged enzyme as shown in Figure 1 followed by filtration. The second drawing shows a process of preparing Ubstac enzyme aggregate by reacting the Ubstac mixture with a ubiquitin C-terminal tagged enzyme, followed by precipitation with a cross linker. The third drawing schematically shows a process of immobilizing the ubiquitin C terminal tagged protein onto a substrate or a bead.

### Preparation Example 3: Preparation of Ubstac using only E1-E2 (E2 platform)

The E2-Ubstac was prepared by using E2-25K (GenBank ID-U58522.1) (human E2), Ucb13 (yeast E2)-MMS2 (GenBank ID-U66724.1) (yeast ubiquitin-conjugating enzyme variant) (GenBank ID-U66724.1). The recombinant DNA plasmid was requested to be synthesized by Genscript. The E2-Ubstac reaction (a total volume of 50 µL) was carried out under a condition of the E2-Ubstac buffer (50 mM Tris pH 8.0, 5 mM MgCl₂), and the E2-Ubstac mixture (1 uM E1, 10 uM E2, 4 mM ATP) was added to the free ubiquitin solution (20 µM) to initiate the reaction. The E2-Ubstac reaction was carried out by shaking at room temperature for 1 hour. The results are shown in Figure 5.

### Example

### Example 1: Analysis of activity and stability of xylose reductase (XR)

### 1-(1) Analysis of activity of xylose reductase

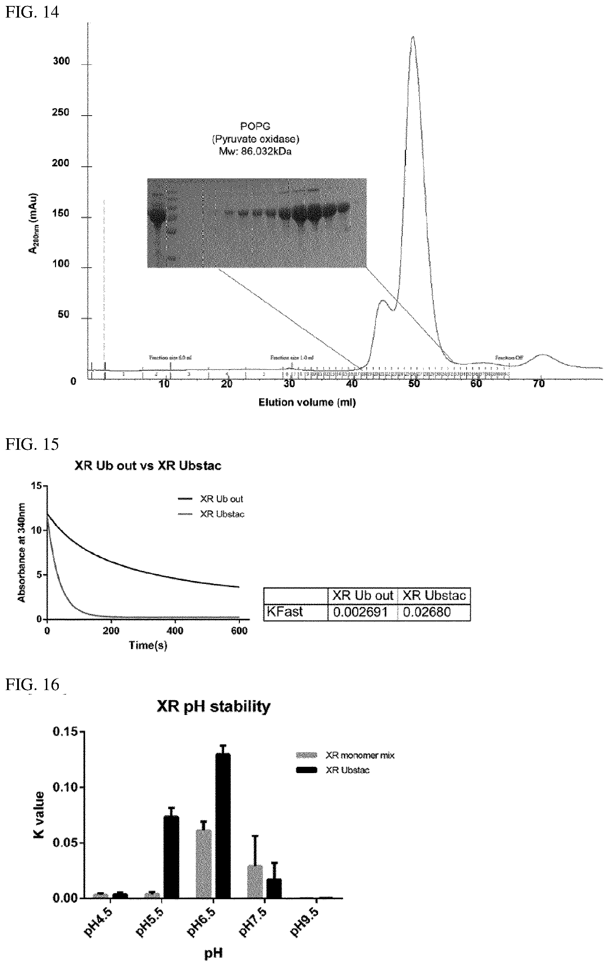

The Ubstac reaction (a total volume of 50 µL) was carried out in the Ubstac buffer (25 mM HEPES pH 7.5, 50 mM NaCl, 4 mM MgCl₂), and the Ubstac mixture (0.5 µM E1, 5 µM E2, 1 µM E3, 4 mM ATP) was added to the XR protein solution to initiate the reaction. The Ubstac reaction was carried out by shaking at room temperature for 1 hour, and then the catalytic activity was analyzed. The catalytic activity of XR was analyzed by measuring the change in absorbance at 340 nm induced by NADH oxidation. The reaction for analysis of the catalytic activity was initiate by adding NADH (2 mM) to a mixture of XR (10 uM) and xylose (200 mM) in 100 mM NaCl buffer (pH 7.0) containing 1 mM MgCl₂ and 0.02% Tween-20. XR ub out was a sample in the form of a monomer that did not comprise a ubiquitin tag at the C-terminus of the XR, and did not form a polymer under the same Ubstac mixing condition. The statistical analysis was carried out using Prism 6 (GraphPad Software, Inc). The results are shown in Figure 15. As shown in Figure 15, the XR according to the present invention promoted the reduction of D-xylose to xylitol by using NADH as a co-substrate. Absorbance represents the amount of NADH in solution. The Ubstac polymer of the XR (red, lower curve) showed faster NADH consumption compared to the monomer form (black, upper curve). Both reactions contained the same amount of the monomers. Therefore, the increased reaction rate is solely dependent on the covalent bonds between the monomers. In this example, it was confirmed that the activity of the XR Ubstac polymer was increased by 10 times compared to the XR monomer without ubiquitin-tag (XR Ub out).

### 1-(2) Analysis of pH stability of xylose reductase

Both the XR monomer and the Ubstac polymer were treated for 30 minutes at the indicated pH before initiating the reaction with the addition of NADH and xylose. As shown in Figure 16, at pH 5.5 and 6.5, the XR Ubstac polymer showed significantly enhanced stability compared to the XR monomer without ubiquitin-tag (XR Ub out). The results represent the average value of the three experiments.

### Example 2: Analysis of activity and stability of oxaloacetate decarboxylase (OAC)

### 2-(1) Analysis of activity of OAC

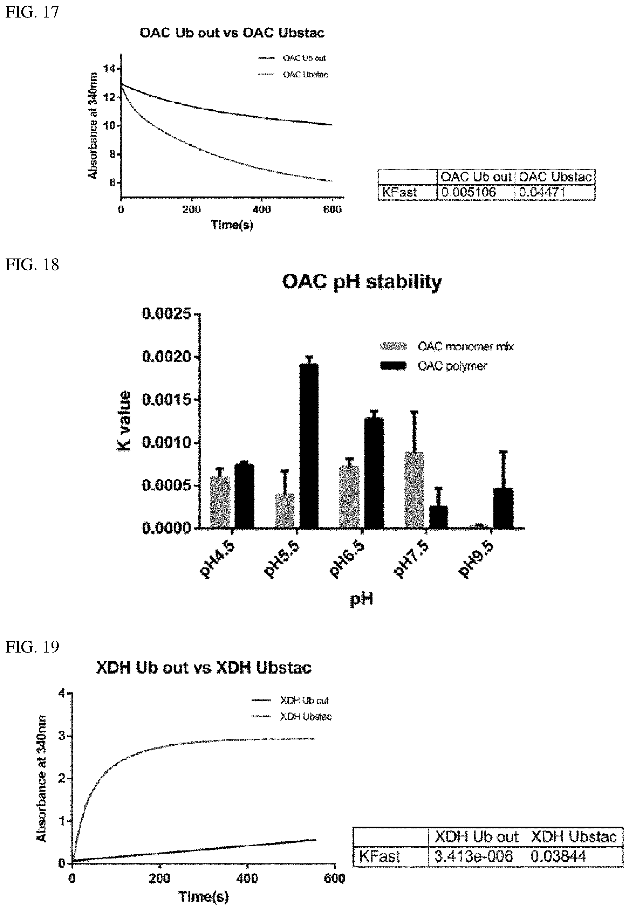

OAC involved in gluconeogenesis is used to investigate liver damage in conjunction with AST-ALT. The Ubstac reaction (a total volume of 50 µL) was carried out in the Ubstac buffer (25 mM HEPES pH 7.5, 50 mM NaCl, 4 mM MgCl₂), and the Ubstac mixture (0.5 µM E1, 5 µM E2, 1 µM E3, 4 mM ATP) was added to the OAC protein solution to initiate the reaction. The Ubstac reaction was carried out by shaking at room temperature for 1 hour, and then the catalytic activity was analyzed. The analysis of OAC activity was based on the decrease in absorbance (340 nm) as NADH consumption proceeded under the following conditions: 45 mM TEA buffer pH 8.0, 0.45 mM MnCl₂, 2 mM NADH, 11 U of LDH, 5 µM OAC, 2.5 mM. The OAC Ubout was a sample in the form of a monomer that did not comprise a ubiquitin-tag at the C-terminus of the OAC, and did not form a polymer under the same Ubstac mixing condition. The statistical analysis was carried out using Prism 6 (GraphPad Software, Inc). The results are shown in Figure 17. As shown in Figure 17, as a result of comparing the activity of the monomer (OAC Ub out) without Ub at the C-terminus and the activity of the polymer (OAC Ubstac), the activity of the polymer was increased by 9 times. Absorbance represents the amount of NADH in solution. The Ubstac polymer of the OAC (red, lower curve) showed faster NADH consumption compared to the monomer form (black, upper curve). Both reactions contained the same amount of the monomers. Therefore, the increased reaction rate is solely dependent on the covalent bonds between the monomers. In this example, it was confirmed that the activity of the OAC Ubstac polymer was increased by 9 times compared to the OAC monomer without ubiquitin-tag (OAC Ub out).

### 2-(2) Analysis of stability of OAC

Both the OAC monomer and the Ubstac polymer were treated for 30 minutes at the indicated pH before initiating the reaction with the addition of NADH and oxaloacetate. As shown in Figure 18, at a low pH of pH 4.5 to 6.5, the OAC Ubstac polymer showed significantly enhanced pH stability compared to the OAC monomer without ubiquitin-tag (OAC Ub out). The results represent the average value of the three experiments.

### Example 3: Analysis of activity and stability of xylitol dehydrogenase (XDH)

### 3-(1) Analysis of activity of XDH

XDH is an enzyme belonging to the D-Xylose catabolism pathway, and is known to convert xylitol, a product of XR, into xylulose using NAD+. For analysis of activity of XDH, the Ubstac reaction (a total volume of 50 µL) was first carried out in the Ubstac buffer (25 mM HEPES pH 7.5, 50 mM NaCl, 4 mM MgCl₂), and the Ubstac mixture (0.5 µM E1, 5 µM E2, 1 µM E3, 4 mM ATP) was added to the XDH protein solution to initiate the reaction. The Ubstac reaction was carried out by shaking at room temperature for 1 hour, and then the catalytic activity was analyzed. The activity of XDH was measured by monitoring NAD+ reduction at 340 nm. The reaction was initiated by adding NADH (2 mM) to a mixture of XDH (20 µM) and xylose (200 mM) in 100 mM NaCl buffer (pH 7.0) containing 1 mM MgCl₂ and 0.02% Tween-20. The XDH ub out was a sample in the form of a monomer that did not comprise a ubiquitin-tag at the C-terminus of the XDH, and did not form a polymer under the same Ubstac mixing condition. The statistical analysis was carried out using Prism 6 (GraphPad Software, Inc). The results are shown in Figure 19. As shown in Figure 19, at pH 5.5, the Ubstac polymer of the XDH (red, upper curve) showed higher NADH+ consumption rate compared to the monomer form (black, lower curve). Both reactions contained the same amount of the monomers. Therefore, the difference in activity is solely dependent on the covalent bonds between the monomers. In this example, it was confirmed that the activity of the XDH Ubstac polymer was increased by 10 times compared to the OAC monomer without ubiquitin-tag (OAC Ub out).

### 3-(2) Analysis of stability of XDH

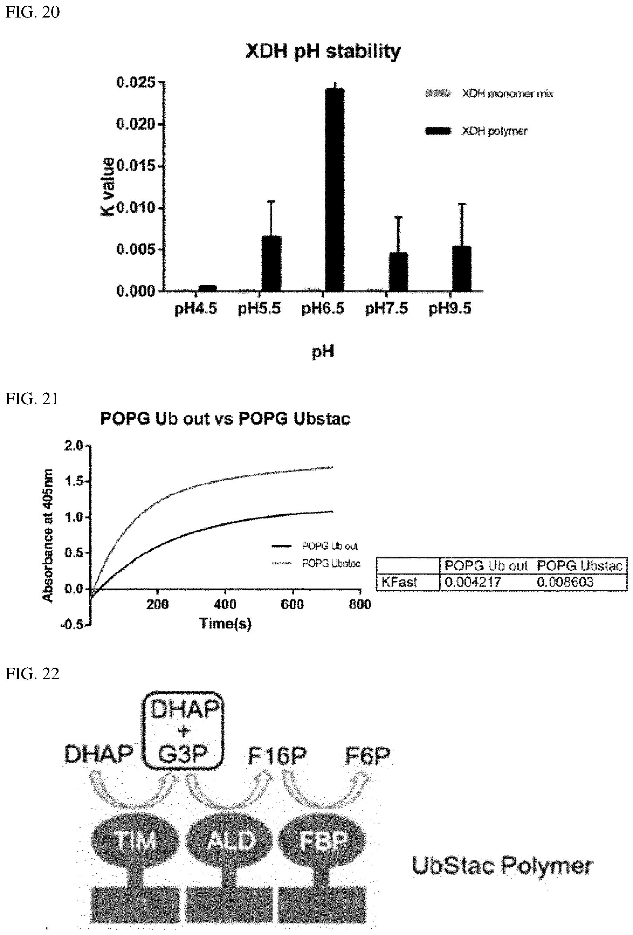

Both the XDH monomer and the Ubstac polymer were treated for 30 minutes at the indicated pH before initiating the reaction with the addition of NAD+ and xylitol. As shown in Figure 20, at all measured pHs, the XR Ubstac polymer showed significantly increased pH stability compared to the OAC Ub out. The results represent the average value of the three experiments.

### Example 4: Analysis of activity of pyruvate oxidase (POPG)

POPG is known to be used to investigate liver damage by detecting enzymes such as AST-ALT, an enzyme involved in the gluconeogenesis process. For analysis of activity of POPG, the Ubstac reaction (a total volume of 50 µL) was first carried out in the Ubstac buffer (25 mM HEPES pH 7.5, 50 mM NaCl, 4 mM MgCl₂), and the Ubstac mixture (0.5 uM E1, 5 uM E2, 1 uM E3, 4 mM ATP) was added to the POPG protein solution to initiate the reaction. The Ubstac reaction was carried out by shaking at room temperature for 1 hour, and then the catalytic activity was analyzed. In order to analyze the catalytic activity, the amount of H₂O₂ produced by the POPG oxidation process of pyruvate by ABTS was measured. The reaction was initiated by adding POPG (5 µM) to a mixture of pyruvate (100 mM), pyrophosphate (6 mM), ABTS (2,2'-azino-bis(3-ethylbenzothiazoline-6-sulfonic acid) (10 mM) and HRP (horseradish peroxidase) (0.2 U/mL) in sodium phosphate buffer. The POPG monomer (POPG ub out) was a sample in the form of a monomer that did not comprise a ubiquitin tag at the C-terminus of the POPG, and did not form a polymer under the same Ubstac mixing condition. The statistical analysis was carried out using Prism 6 (GraphPad Software, Inc). As shown in Figure 21, at pH 5.5, the POPG (red, upper curve) showed higher activity compared to its monomer form (black, lower curve). Both reactions contained the same amount of the monomers. Therefore, the difference in activity is solely dependent on the covalent bonds between the monomers. In this particular example, the activity of the POPG UbStac polymer was increased by 2 times compared to the POPG monomer without ubiquitin-tag (POPG Ub out).

### Example 5: Analysis of synergistic effect of ubiquitin enzyme

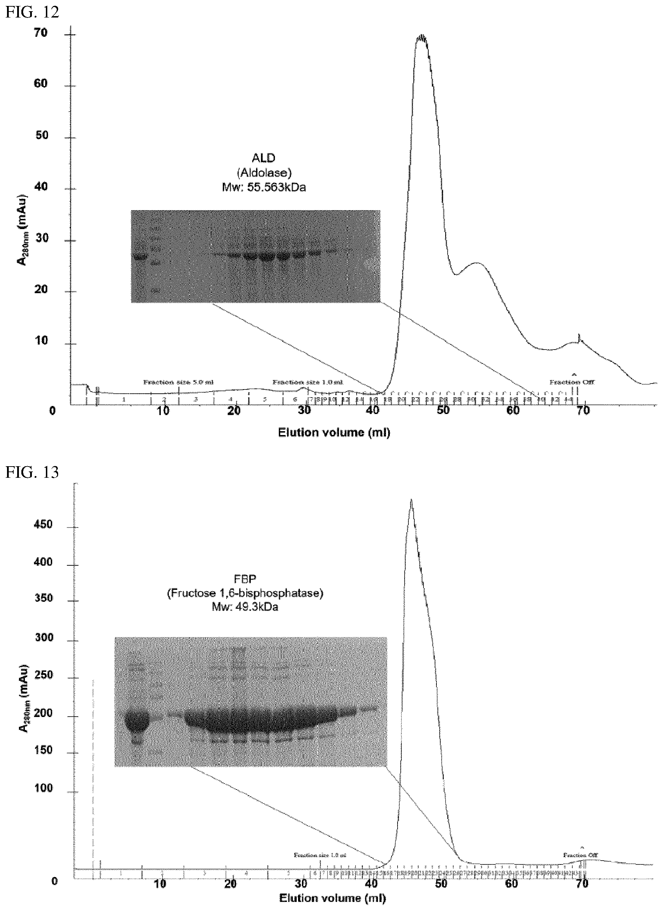

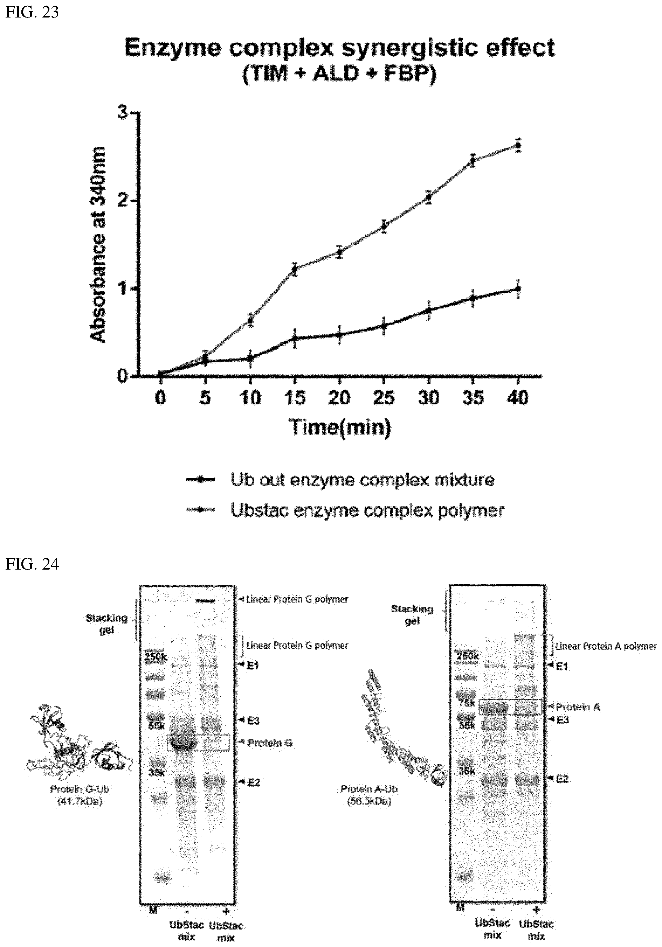

Triosephosphate isomerase (TIM), fructose bisphosphate aldolase (ALD) and fructose bisphosphatase (FBP) are known to form a cascade reaction for producing F6P as a final product from DHAP (dihydroxyacetone phosphate). The analysis of synergistic effect of the Ubstac enzyme was carried out by measuring Fructose-6-Phosphate (F6P), TIM product, ALD and FBP enzyme complex. F6P is isomerized to glucose-6-phosphate (G6P) by phosphoglucose isomerase (PGI), and the same amount of NAD+ as a substrate is modified by glucose-6-phosphate dehydrogenase (G6PDH). The present inventors determined the enzyme activity by measuring the amount of newly generated NADH by adding 2.5 mM of enzyme complex (dihydroxyacetone phosphate, DHAP), 20 U/mL analysis enzyme (PGI and G6PDH) and 2.5 mM NAD+ enzyme complex to a mixture of 4 uM TIM, ALD and FBP enzyme complex in a HEPES buffer condition (200 mM HEPES pH 7.5, 10 mM MgCl₂, 0.5 mM MnCl₂, 1 mM CaCl₂) at 340 nm. The Ub out enzyme complex mixture was a sample in the form of a monomer that did not comprise a ubiquitin tag at the C-terminus of the enzyme, and did not form a polymer under the same Ubstac mixing condition. The statistical analysis was carried out using Prism 6 (GraphPad Software, Inc). At the indicated time point, the reaction was terminated, and the amount of F6P was measured using a phosphoglucose isomerase (PGI) that uses NAD+ to convert F6P into glucose-6-phosphate (G6P). Absorbance represents the amount of F6P. The Ubstac polymer of three different enzymes (red, upper curve) showed higher activity by five times than the monomeric enzyme mixture (black, lower curve). The results represent the average value of the three experiments. Figure 23 shows the resulting Ubstac product of the structure in which three enzymes, TIM, ALD and FBP are bound, and Figure 12 shows the synergistic effect by these enzymes.

### Example 9: Ubiquitin multistage labeling (prosthetics) method

First, a ubiquitin C-terminal tagged biomolecule was synthesized according to the Preparation Examples of the present invention. Next, a polymer (polyethylene glycol) comprising hydroxylamine was reacted with the above biomolecule. As a result, it was confirmed that the polymer was labeled by ubiquitin by oxime linking (the results are not shown).

### Example 10: Preparation of Protein A and Protein G linear multimer polymer

The Ubstac reaction (a total volume of 50 µL) was carried out in the Ubstac buffer (25 mM HEPES pH 7.5, 50 mM NaCl, 4 mM MgCl₂), and the Ubstac mixture (0.5 µM E1, 5 µM E2, 1 µM E3, 4 mM ATP) was added to the Protein A or Protein G solution to initiate the reaction. Recombinant DNA plasmids comprising sequences corresponding to Protein A (GenBank ID-AAB05743.1) and Protein G (CAA27638.1) were requested to be synthesized by Genscript. The Ubstac reaction was carried out by shaking at room temperature for 1 hour, and then SDS-PAGE was carried out. Compared with the sample without the Ubstac mixture, it was confirmed that the Protain A or Protein G monomer band was reduced in the sample to which the Ubstac mixture was added, and a band of high molecular weight (linear multimer polymer) newly appeared (see Figure 24). It was confirmed that some linear multimer polymers did not pass through a stacking gel due to an increase in molecular weight of up to several hundreds kDa.

### Example 11: hGH in which C-terminus of the glycine at the 76th amino acid residue of ubiquitin C-terminal tag is extended by aspartate

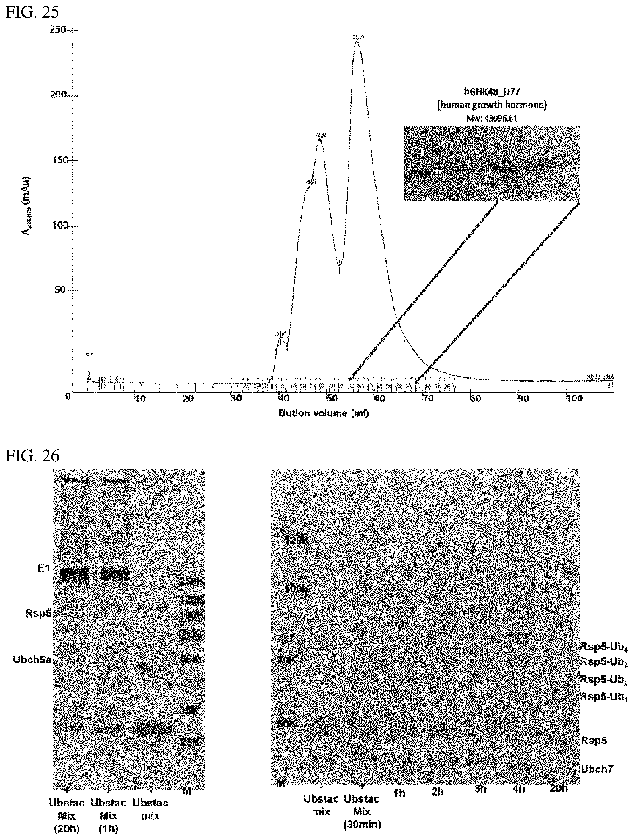

For overexpression of protein for UCT fusion drug, each gene construct was transformed into *E. coli* BL21 DE3 (Novagen) strain. In this example, hGH (SEQ ID NO: 18) was used as a protein. Cells comprising the protein expression plasmid (pET21a, Genscript) were incubated in LB medium (Miller) at 37 °C. When the OD₆₀₀ value reached about 0.6, the protein expression was induced with 250 µM of isopropyl β-D-1-thiogalactopyranoside (isopropyl-beta-D-thiogalactopyranoside) (IPTG) at 16 °C for 20 hours. Next, after centrifugation (at 3,500 rpm at 4 °C for 15 minutes), cell pellet was resuspended in lysis buffer (20 mM Tris-HCl pH 8.0, 500 mM NaCl₂, 20 mM imidazole), and lysed by sonication (50% amplitude, pluse on 3 seconds-off 5 seconds, final 15 minutes). Then, the lysate was further centrifuged at 14,000 rpm at 4 °C for 30 minutes. The water soluble fraction of the protein comprising the N-terminal His-tag was purified by gel filtration chromatography using Superdex 75 pg gel filtration column 16/600 (GE Healthcare) pre-equilibrated with nickel affinity and FPLC buffer (Ni-NTA Agarose - QIAGEN, 20 mM Tris-HCl pH 8.0, 150 mM NaCl₂). The purified hGH was concentrated to 100 µM. All target proteins were evaluated by SDS-PAGE (see Figure 25).

### Example 12: Preparation of polvubiquitin scaffold originated from E3 (Rsp5)

The Ubstac reaction (a total volume of 50 µL) was carried out in the Ubstac buffer (25 mM HEPES pH 7.5, 50 mM NaCl, 4 mM MgCl₂), and the Ubstac mixture (0.5 µM E1, 5 µM E2 (Ubch5a or Ubch7), 1 uM E3, 4 mM ATP) was added to the protein solution to initiate the reaction. The Ubstac reaction was carried out by shaking at room temperature for 1 hour, and SDS-PAGE was carried out. It was confirmed that the amount of E3 was reduced in the sample to which Ubstac was added compared to the sample without the Ubstac mixture (see Figure 26). This is because the band of E3 whose molecular mass was increased due to the formation of a polyubiquitin scaffold originated from E3 was shifted upwards. In the results of using Ubch7 E2, which is less reactive than Ubch5a E2, it was confirmed that the molecular weight was increased by adding ubiquitin one by one to Rsp5 over time.

### Example 13: Preparation of polymer of hGH expressed in extended form with aspartate at C-terminus of UCT repeatedly linked in head-to-tail form

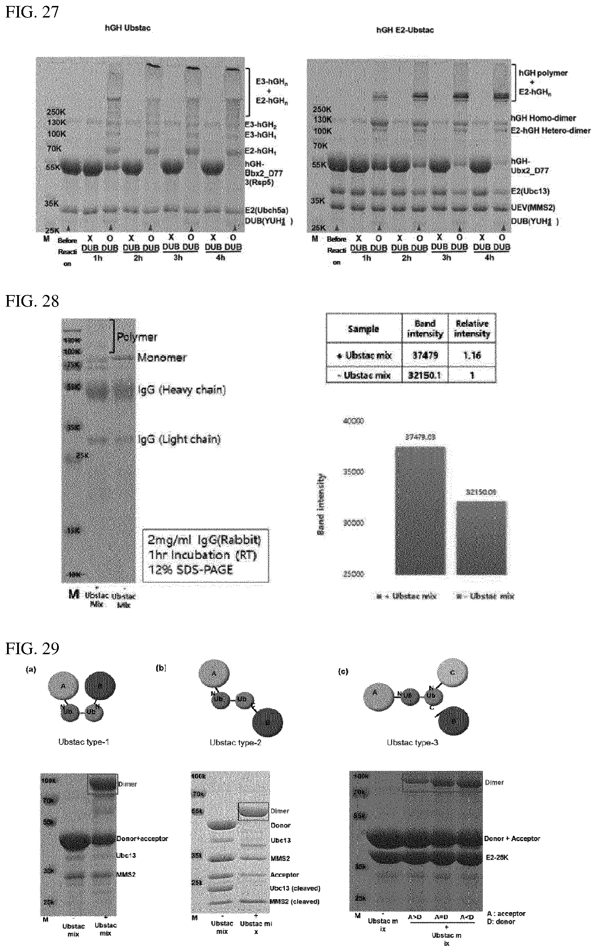

As shown in Figure 27, the Ubstac reaction of hGH (SEQ ID NO: 18) was compared under the condition where DUB was present together and the condition where DUB was excluded. The hGH used at this time is one in which two ubiquitin tags are repeatedly connected at the C-terminus in a head-to-tail form, and the C-terminus of the ubiquitin tag is extended with aspartate. Therefore, if the aspartate at the C-terminus of the ubiquitin tag is not cleaved using DUB, the Ubstac reaction does not occur. The Ubstac reaction to confirm the polymer formation of hGH, a biomolecule for drug (a total volume of 50 µL) was carried out in the Ubstac buffer (25 mM HEPES pH 7.5, 50 mM NaCl, 4 mM MgCl₂), and the Ubstac mixture (1 µM E1, 5 µM E2 (ubch5a), 1 µM E3, 4 mM ATP) was added to 20 µM hGH protein solution to initiate the reaction. The E2-Ubstac reaction where E3 was excluded (a total volume of 50 µL) was carried out in the E2-Ubstac buffer (50 mM Tris pH 8.0, 5 mM MgCl₂), and the E2-Ubstac mixture (1 µM E1, 10 µM E2 (Ucb13-MMS2 complex), 4 mM ATP) was added to 20 µM hGH protein solution to initiate the reaction. In order to confirm the activity of DUB together, the reaction was carried out simultaneously under the condition where DUB (YUH1) was absent and the condition where DUB (YUH1) was present at a concentration of 2 µM, respectively. All reactions were carried out by shaking at room temperature for 1 to 4 hours, and then confirmed by SDS-PAGE. As shown in Figure 27, it was confirmed that the polymer of hGH was formed only under the condition where DUB was present, and it was confirmed that the polymer was not formed because the aspartate at the C-terminus of hGH UCT was not cleaved under the condition where DUB was absent.

### Example 14: Preparation of polymer of hGH expressed in extended form with aspartate at C-terminus of UCT repeatedly linked in head-to-tail form

The Ubstac reaction (a total volume of 50 µL) to prepare the Protein A polymer was carried out in the Ubstac buffer (25 mM HEPES pH 7.5, 50 mM NaCl, 4 mM MgCl₂). The Ubstac mixture (0.5 µM E1, 5 µM E2 (Ubch5a or Ubch7), 1 µM E3, 4 mM ATP) was added to the Protein A protein solution to initiate the reaction. The Ubstac reaction was carried out by shaking at room temperature for 1 hour, and then mixed in a 1:1 ratio with latex beads at 50% concentration, and then shaken at ambient temperature for 4 hours, and the reaction to immobilize the Protein A polymer on the beads was carried out. After the immobilization reaction, in order to remove the unimmobilized protein, washing was carried out three times with PBS buffer (10 mM Na₂HPO₄ pH 7.4, 1.8 mM KH₂PO₄, 137 mM NaCl, 2.7 mM KC1). After washing, the immunoglobulin G (IgG) obtained from human serum was added to the beads at a concentration of 2 mg/mL to analyze the binding activity of the Protein A polymer immobilized on the beads. The binding reaction was carried out by shaking at ambient temperature for 1 hour, and then washed three times with PBS buffer in the same manner as in the above washing method, and then confirmed by SDS-PAGE. As a result, it was confirmed that the binding activity of human derived IgG to the bead on which the protein A polymer was immobilized was increased by 15% or more compared to the bead on which the protein A monomer was immobilized by proceeding the same without adding the Ubstac mix (see Figure 28).

### Example 15: Preparation of linear multivalent biomolecule polymer bound to N-terminus, C-terminus, or both N-terminus and C-terminus of ubiquitin, respectively

The formation of the Ubstac dimer was confirmed using the donor ubiquitin in which hGH (SEQ ID NO: 18) was bound to the N-terminus, the acceptor ubiquitin (Figure 29 (a)) in which hGH was bound to the N-terminus, the acceptor ubiquitin (Figure 29 (b)) in which hGH was bound to the C-terminus, and the acceptor ubiquitin (Figure 29 (c)) in which sumo (SEQ ID NO: 19) and hGH were bound to the N-terminus and the C-terminus, respectively. The used acceptor ubiquitin is a form in which the leucine at the 73rd amino acid residue is substituted with proline, and other lysines except for the lysine at the 48th (Figure 29 (c)) or the 63rd (Figure 29 (a), (b)) amino acid residue are substituted with arginine, and the C-terminus is extended by aspartate or biomolecule (hGH). The Ubstac reaction (Figure 29 (a), (b)) (a total volume of 50 µL) was carried out in the Ubstac buffer (25 mM HEPES pH 7.5, 50 mM NaCl, 4 mM MgCl₂), and the Ubstac mixture (1 uM E1, 5 uM E2 (Ubc13-MMS2 complex), 4 mM ATP) was added to a solution (a total ubiquitin concentration of 20 uM) in which 10 uM acceptor ubiquitin protein and donor ubiquitin protein were mixed to initiated the reaction. The Ubstac reaction (Figure 29 (c)) was initiated by replacing E2 with E2-25K, not Ubc13-MMS2 complex, and the acceptor ubiquitin with a protein having only the lysine at the 48^{th} amino acid residue other than the lysine at the 63rd amino acid residue under the same conditions as the above reaction. The Ubstac reaction was carried out by shaking at 27°C for 4 hours, and then confirmed by SDS-PAGE. In the Ubstac reaction (Figure 29 (b)), the acceptor ubiquitin in the Ub-hGH form in which His-sumo was cleaved using the SENP1 enzyme from a protein in the His-sumo-Ub-hGH form was used, and it was confirmed that at this time, the remaining SENP1 was included in the Ubstac reaction, and thus the donor hGH, Ubc13 and His-sumo of MMS2 were also cleaved together, and the band of dimer and E2 (Ubc13, MMS2) after reaction was shifted. As shown in Figure 29, it was confirmed that the Ubstac dimer was formed in all forms in which the biomolecules were bound to the N-terminus, the C-terminus, or both of the N-terminus and the C-terminus, respectively. SEQ ID NO of proteins and the like used in this example are as follows: the donor ubiquitin in which hGH was bound to the N-terminus (SEQ ID NO: 20); the acceptor ubiquitin in which hGH was bound to the N-terminus (SEQ ID NO: 21); the acceptor ubiquitin in which hGH was bound to the C-terminus (SEQ ID NO: 22); and the acceptor ubiquitin in which SUMO was bound to the N-terminus and hGH was bound to the C-terminus (SEQ ID NO: 23).

### Industrial Availability

The present invention relates to a method for preparing a linear multimeric biomolecule polymer in which ubiquitin C-terminal tag (UCT)-biomolecule is a unit in vitro using the E1-E2-E3 system involved in the ubiquitin-proteasome proteolysis in vivo, and the linear multimeric biomolecule polymer prepared by this method. As described above, the linear multimeric biomolecule polymer of the present invention and a method for preparing the same can be widely used in industrial and medical fields requiring production of immobilized proteins, separation and purification of substances, and analysis.

## Claims

1. A method for preparing a linear polyfunctional multimeric biomolecule, wherein the method comprises
(i) recombinantly expressing a biomolecule to which a ubiquitin C-terminal tag is fused or bound by a linker from a host cell including a prokaryotic cell or a eukaryotic cell, and
(ii) adding E1, E2 and E3 enzymes for ubiquitination, or E1 and E2 enzymes for ubiquitination to a cell lysate of the host cell and reacting them,
wherein the biomolecule is bound to a polyubiquitin scaffold formed of two or more covalently bonded ubiquitins, and the biomolecule comprises two or more binding moieties, each specific for different binding sites.

2. The method according to claim 1, wherein the E2 enzyme binds to the lysine at the 48th or 63rd amino acid residue of ubiquitin.

3. The method according to claim 2, wherein the E2 enzyme is an E2-25K ubiquitin conjugating enzyme.

4. The method according to claim 2, wherein the E2 enzyme is Ucb13-MMS2, a ubiquitin conjugating enzyme complex.

5. The method according to claim 1 or 2, wherein the biomolecule has active sites that specifically bind to other biomolecules, small molecule chemical compounds or nanoparticles or the like, and is one or more selected from the group consisting of an enzyme, a protein, a peptide, a polypeptide, an antibody, an antibody fragment, DNA and RNA.

6. The method according to claim 5, wherein the biomolecule is one or more selected from the group consisting of protein A, protein G, lysin, endolysin, protease, hydrolase, oxidoreductase, lyase, affinity ligand and receptor.

7. The method according to claim 1 or 2, wherein the biomolecule is selected from the group consisting of insulin, insulin analogue, glucagon, glucagon-like peptides (GLP-1 and the like), GLP-1/glucagon dual agonist, exendin-4, exendin-4 analogue, insulin secreting peptide and an analogue thereof, human growth hormone, growth hormone releasing hormone (GHRH), growth hormone releasing peptide, granulocyte colony stimulating factor (G-CSF), anti-obesity peptide, G-protein-coupled receptor, leptin, GIP (gastric inhibitory polypeptide), interleukins, interleukin receptors, interleukin binding proteins, interferons, interferon receptors, cytokine binding proteins, macrophage activator, macrophage peptide, B cell factor, T cell factor, suppressive factor of allergy, cell necrosis glycoprotein, immunotoxin, lymphotoxin, tumor necrosis factor (TNF), tumor inhibitory factor, metastasis growth factor, alpha-1 antitrypsin, albumin, α-lactalbumin, apolipoprotein-E, erythropoietin (EPO), high glycosylated erythropoietin, angiopoietins, hemoglobin, thrombin, thrombin receptor activating peptide, thrombomodulin, blood factors VII, VIIa, VIII, IX, and XIII, plasminogen activator, fibrin-binding peptide, urokinase, streptokinase, hirudin, protein C, C-reactive protein, renin inhibitor, collagenase inhibitor, superoxide dismutase, platelet derived growth factor, epithelial growth factor, epidermal growth factor, angiostatin, angiotensin, bone formation growth factor, bone formation promoting protein, calcitonin, atriopeptin, cartilage inducing factor, elcatonin, connective tissue activator, tissue factor pathway inhibitor, follicle stimulating hormone (FSH), luteinizing hormone (LH), luteinizing hormone releasing hormone (LHRH), nerve growth factors, parathyroid hormone (PTH), relaxin, secretin, somatomedin, adrenal cortical hormone, cholecystokinin, pancreatic polypeptide, gastrin releasing peptide, corticotropin releasing factor, thyroid stimulating hormone (TSH), autotaxin, lactoferrin, myostatin, receptor, receptor antagonist, fibroblast growth factor, adiponectin, interleukin receptor antagonist, cell surface antigen, virus derived vaccine antigen, monoclonal antibody, polyclonal antibody and antibody fragments.

8. The method according to claim 1 or 2, wherein the recombinantly expressed biomolecule is one in which a C-terminal portion of the glycine at the 76th amino acid residue from the N-terminus of the ubiquitin, which is a ubiquitin C-terminal tag, is extended by 1 to 50 amino acids, and the method further comprises adding DUB (deubiquitinating enzyme) to the recombinantly expressed biomolecule before or after the reaction of step (ii).

9. The method according to claim 8, wherein the biomolecule is extended by aspartate, 6xHis, chitin binding domain, GST, thrombin, FLAG tag.

10. The method according to claim 8 or 9, wherein the DUB is YUH1, YUH2, UCH-L1, UCH-L2 or UCH-L3.

11. The method according to claim 1 or 2, wherein the ubiquitin C-terminal tag is one in which other lysines except for one lysine at any position thereof are deleted or substituted with amino acids other than lysine.

12. The method according to claim 11, wherein all lysines of the ubiquitin except for the lysine at the 11th, 48th, or 63rd amino acid residue starting from the N-terminal Met1 of the ubiquitin are substituted with arginine.

13. The method according to claim 1 or 2, wherein the ubiquitin C-terminal tag is one in which two or more ubiquitins are repeatedly linked in a head-to-tail form.

14. The method according to claim 13, wherein the ubiquitin linked in the head-to-tail form is one in which the glycines at the 75th and 76th amino acid residues from the N-terminus are substituted with other amino acids including valine.

15. The method according to claim 13 or 14, wherein the leucine at the 73rd residue from the N-terminus of the ubiquitin is substituted with proline.

16. A linear polyfunctional multimeric biomolecule polymer comprised of a polyubiquitin scaffold and a biomolecule, wherein the linear polyfunctional biomolecule polymer comprises two or more binding moieties that are specific for different binding sites, and the polyubiquitin scaffold is formed of two or more covalently bonded ubiquitins; the biomolecule has active sites that specifically bind to other biomolecules, small molecule chemical compounds or nanoparticles or the like, and the biomolecule is bound to the N-terminus, the C-terminus, or both the N-terminus and the C-terminus of the ubiquitin.

17. The linear polyfunctional multimeric biomolecule polymer according to claim 16, wherein the linear multimeric biomolecule polymer is comprised of 2 to 4 biomolecules.

18. The linear polyfunctional multimeric biomolecule polymer according to claim 16 or 17, wherein the biomolecule is bound by a linker to the N-terminus, the C-terminus, or both the N-terminus and the C-terminus of the ubiquitin.

19. The linear polyfunctional multimeric biomolecule polymer according to claim 18, wherein the linker is a combination of 1 to 6 repeats of GGGGS or EAAAK

20. The linear polyfunctional multimeric biomolecule polymer according to claim 16, wherein the biomolecule bound to the N-terminus of the ubiquitin is the distal end of the linear multimeric biomolecule polymer.

21. The linear polyfunctional multimeric biomolecule polymer according to claim 16, wherein the biomolecule bound to the C-terminus, the N-terminus, or both the C-terminus and the N-terminus of the ubiquitin is the proximal end of the linear multimeric biomolecule polymer.

22. The linear polyfunctional multimeric biomolecule polymer according to claim 16 or 17, wherein the polyubiquitin scaffold is formed by covalently linking a donor ubiquitin in which all lysines of the ubiquitin are substituted with arginine, and an acceptor ubiquitin in which all lysines of the ubiquitin except for the lysine at the 11th, 48th, or 63rd amino acid residue from the N-terminus are substituted with arginine.

23. The linear polyfunctional multimeric biomolecule polymer according to claim 16 or 17, wherein the leucine at the 73^{rd} amino acid residue from the N-terminus of the ubiquitin is substituted with proline.

24. The linear polyfunctional multimeric biomolecule polymer according to claim 22, wherein the acceptor ubiquitin is extended by aspartate, 6xHis tag, or GST tag.

25. The linear polyfunctional multimeric biomolecule polymer according to claim 16, wherein the linear multimeric biomolecule polymer is comprised of 2 to 20 biomolecules.

26. The linear polyfunctional multimeric biomolecule polymer according to claim 16 or 17, wherein the biomolecule is one or more selected from the group consisting of an enzyme, a protein, a peptide, a polypeptide, an antibody, an antibody fragment, DNA and RNA.

27. The linear polyfunctional multimeric biomolecule polymer according to claim 18, wherein the biomolecule is one or more selected from the group consisting of protein A, protein G, lysin, endolysin, protease, hydrolase, oxidoreductase, lyase, affinity ligand and receptor.

28. The linear polyfunctional multimeric biomolecule polymer according to claim 16 or 17, wherein the biomolecule is selected from the group consisting of insulin, insulin analogue, glucagon, glucagon-like peptides (GLP-1 and the like), GLP-1/glucagon dual agonist, exendin-4, exendin-4 analogue, insulin secreting peptide and an analogue thereof, human growth hormone, growth hormone releasing hormone (GHRH), growth hormone releasing peptide, granulocyte colony stimulating factor (G-CSF), anti-obesity peptide, G-protein-coupled receptor, leptin, GIP (gastric inhibitory polypeptide), interleukins, interleukin receptors, interleukin binding proteins, interferons, interferon receptors, cytokine binding proteins, macrophage activator, macrophage peptide, B cell factor, T cell factor, suppressive factor of allergy, cell necrosis glycoprotein, immunotoxin, lymphotoxin, tumor necrosis factor (TNF), tumor inhibitory factor, metastasis growth factor, alpha-1 antitrypsin, albumin, α-lactalbumin, apolipoprotein-E, erythropoietin (EPO), high glycosylated erythropoietin, angiopoietins, hemoglobin, thrombin, thrombin receptor activating peptide, thrombomodulin, blood factors VII, VIIa, VIII, IX, and XIII, plasminogen activator, fibrin-binding peptide, urokinase, streptokinase, hirudin, protein C, C-reactive protein, renin inhibitor, collagenase inhibitor, superoxide dismutase, platelet derived growth factor, epithelial growth factor, epidermal growth factor, angiostatin, angiotensin, bone formation growth factor, bone formation promoting protein, calcitonin, atriopeptin, cartilage inducing factor, elcatonin, connective tissue activator, tissue factor pathway inhibitor, follicle stimulating hormone (FSH), luteinizing hormone (LH), luteinizing hormone releasing hormone (LHRH), nerve growth factors, parathyroid hormone (PTH), relaxin, secretin, somatomedin, adrenal cortical hormone, cholecystokinin, pancreatic polypeptide, gastrin releasing peptide, corticotropin releasing factor, thyroid stimulating hormone (TSH), autotaxin, lactoferrin, myostatin, receptor, receptor antagonist, fibroblast growth factor, adiponectin, interleukin receptor antagonist, cell surface antigen, virus derived vaccine antigen, monoclonal antibody, polyclonal antibody and antibody fragments.

29. The linear polyfunctional multimeric biomolecule polymer according to claim 16 or 17, wherein the linear multimeric biomolecule polymer is originated from E3, E2, E1, a free ubiquitin, or a substrate.

30. The linear polyfunctional multimeric biomolecule polymer according to claim 29, wherein the E3 is Rsp5, WWP1, nedd4 or XIAP, or a minimal catalytic domain thereof, and the E2 is Ubc7, Ubch5a, E2-25K, Ubc13-MMS2 complex, or a catalytic domain thereof.

31. The linear polyfunctional multimeric biomolecule polymer according to claim 30, wherein the free ubiquitin is one in which other lysines except for one lysine at any position thereof are deleted or substituted with amino acids other than lysine.

32. The linear polyfunctional multimeric biomolecule polymer according to claim 31, wherein the free ubiquitin is one in which all lysines except for the lysine at the 11th, 48th, or 63rd amino acid residue from the N-terminus thereof are substituted with arginine.

33. The linear polyfunctional multimeric biomolecule polymer according to claim 32, wherein the free ubiquitin is one in which a C-terminal portion of the glycine at the 76th amino acid residue from the N-terminus thereof is extended by 1 to 50 amino acids.

34. The linear polyfunctional multimeric biomolecule polymer according to claim 33, wherein the free ubiquitin is extended by aspartate, 6xHis tag, or GST tag.

35. The linear polyfunctional multimeric biomolecule polymer according to claim 31, wherein the biomolecule is linked to the N-terminal Met1 of the free ubiquitin.

36. The linear polyfunctional multimeric biomolecule polymer according to claim 31, wherein E3, E2, E1, a free ubiquitin or a substrate is attached to one terminus of the biomolecule as an initiator.

37. The linear polyfunctional multimeric biomolecule polymer according to claim 29, wherein the substrate is a protein that comprises an amino acid sequence that recognizes E3 ligase and comprises one or more lysine to which ubiquitin is capable of binding.

38. The linear polyfunctional multimeric biomolecule polymer according to claim 37, wherein the protein has PPPY for Rsp5 or Nedd4-1,2.

39. The linear polyfunctional multimeric biomolecule polymer according to claim 31, wherein the ubiquitin is one in which other lysines except for one lysine at any position thereof are deleted or substituted with amino acids other than lysine.

40. The linear polyfunctional multimeric biomolecule polymer according to claim 16 or 17, wherein the biomolecule is a protein having a molecular weight of 40 to 100 kDa.

41. A polyubiquitin scaffold linker for site-specific binding of two or more biomolecules comprissing two or more binding moieties, each specific for different binding sites, which includes
(i) recombinantly expressing a biomolecule to which a ubiquitin C-terminal tag, a ubiquitin, is fused or bound by a linker from a host cell, and
(ii) adding E1, E2 and E3 enzymes for ubiquitination, or E1 and E2 enzymes for ubiquitination to a cell lysate of the host cell and reacting them.


<210>    1
<211>    76
<212>    PRT
<213>    Artificial Sequence

<220>
<223>    Ubiquitin C-terminal Tag

<400>    1

<210>    2
<211>    506
<212>    PRT
<213>    Artificial Sequence

<220>
<223>    Xylose Reductase

<400>    2
Met Gly His His His His His His Ser Asp Gln Glu Ala Lys Pro Ser
  1               5                  10                  15

Thr Glu Asp Leu Gly Asp Lys Lys Glu Gly Glu Tyr Ile Lys Leu Lys
             20                  25                  30

Val Ile Gly Gln Asp Ser Ser Glu Ile His Phe Lys Val Lys Met Thr
         35                  40                  45

Thr His Leu Lys Lys Leu Lys Glu Ser Tyr Cys Gln Arg Gln Gly Val
     50                  55                  60

Pro Met Asn Ser Leu Arg Phe Leu Phe Glu Gly Gln Arg Ile Ala Asp

65                  70                  75                  80

Asn His Thr Pro Lys Glu Leu Gly Met Glu Glu Glu Asp Val Ile Glu
                85                  90                  95

Val Tyr Gln Glu Gln Thr Gly Gly Met Pro Ser Ile Lys Leu Asn Ser
            100                 105                 110

Gly Tyr Asp Met Pro Ala Val Gly Phe Gly Cys Trp Lys Val Asp Val
        115                 120                 125

Asp Thr Cys Ser Glu Gln Ile Tyr Arg Ala Ile Lys Thr Gly Tyr Arg
    130                 135                 140

Leu Phe Asp Gly Ala Glu Asp Tyr Ala Asn Glu Lys Leu Val Gly Ala
145                 150                 155                 160

Gly Val Lys Lys Ala Ile Asp Glu Gly Ile Val Lys Arg Glu Asp Leu
                165                 170                 175

Phe Leu Thr Ser Lys Leu Trp Asn Asn Tyr His His Pro Asp Asn Val
            180                 185                 190

Glu Lys Ala Leu Asn Arg Thr Leu Ser Asp Leu Gln Val Asp Tyr Val
        195                 200                 205

Asp Leu Phe Leu Ile His Phe Pro Val Thr Phe Lys Phe Val Pro Leu
    210                 215                 220

Glu Glu Lys Tyr Pro Pro Gly Phe Tyr Cys Gly Lys Gly Asp Asn Phe
225                 230                 235                 240

Asp Tyr Glu Asp Val Pro Ile Leu Glu Thr Trp Lys Ala Leu Glu Lys
                245                 250                 255

Leu Val Lys Ala Gly Lys Ile Arg Ser Ile Gly Val Ser Asn Phe Pro
            260                 265                 270

Gly Ala Leu Leu Leu Asp Leu Leu Arg Gly Ala Thr Ile Lys Pro Ser
        275                 280                 285

Val Leu Gln Val Glu His His Pro Tyr Leu Gln Gln Pro Arg Leu Ile
    290                 295                 300

Glu Phe Ala Gln Ser Arg Gly Ile Ala Val Thr Ala Tyr Ser Ser Phe
305                 310                 315                 320

Gly Pro Gln Ser Phe Val Glu Leu Asn Gln Gly Arg Ala Leu Asn Thr
                325                 330                 335

Ser Pro Leu Phe Glu Asn Glu Thr Ile Lys Ala Ile Ala Ala Lys His
            340                 345                 350

Gly Lys Ser Pro Ala Gln Val Leu Leu Arg Trp Ser Ser Gln Arg Gly
        355                 360                 365

Ile Ala Ile Ile Pro Lys Ser Asn Thr Val Pro His Leu Leu Glu Asn
    370                 375                 380

Lys Asp Val Asn Ser Phe Asp Leu Asp Glu Gln Asp Phe Ala Asp Ile
385                 390                 395                 400

Ala Lys Leu Asp Ile Asn Leu Arg Phe Asn Asp Pro Trp Asp Trp Asp

405                         410                         415

Lys Ile Pro Ile Phe Val Gly Gly His His His His His His Met Gln
            420                         425                         430

Ile Phe Val Lys Thr Leu Thr Gly Lys Thr Ile Thr Leu Glu Val Glu
        435                         440                         445

Pro Ser Asp Thr Ile Glu Asn Val Lys Ala Lys Ile Gln Asp Lys Glu
    450                         455                         460

Gly Ile Pro Pro Asp Gln Gln Arg Leu Ile Phe Ala Gly Lys Gln Leu
465                         470                         475                         480

Glu Asp Gly Arg Thr Leu Ser Asp Tyr Asn Ile Gln Lys Glu Ser Thr
                        485                         490                         495

Leu His Leu Val Leu Arg Leu Arg Gly Gly
            500                         505


<210>    3
<211>    542
<212>    PRT
<213>    Artificial Sequence

<220>
<223>    Xylitol dehydrogenase


<400>    3
Met Gly His His His His His His Ser Asp Gln Glu Ala Lys Pro Ser
 1               5                         10                         15

Thr Glu Asp Leu Gly Asp Lys Lys Glu Gly Glu Tyr Ile Lys Leu Lys
            20                         25                         30

Val Ile Gly Gln Asp Ser Ser Glu Ile His Phe Lys Val Lys Met Thr
        35                         40                         45

Thr His Leu Lys Lys Leu Lys Glu Ser Tyr Cys Gln Arg Gln Gly Val
    50                         55                         60

Pro Met Asn Ser Leu Arg Phe Leu Phe Glu Gly Gln Arg Ile Ala Asp
65                         70                         75                         80

Asn His Thr Pro Lys Glu Leu Gly Met Glu Glu Glu Asp Val Ile Glu
                        85                         90                         95

Val Tyr Gln Glu Gln Thr Gly Gly Met Thr Ala Asn Pro Ser Leu Val
            100                         105                         110

Leu Asn Lys Ile Asp Asp Ile Ser Phe Glu Thr Tyr Asp Ala Pro Glu
        115                         120                         125

Ile Ser Glu Pro Thr Asp Val Leu Val Gln Val Lys Lys Thr Gly Ile
    130                         135                         140

Cys Gly Ser Asp Ile His Phe Tyr Ala His Gly Arg Ile Gly Asn Phe
145                         150                         155                         160

Val Leu Thr Lys Pro Met Val Leu Gly His Glu Ser Ala Gly Thr Val
                        165                         170                         175

Val Gln Val Gly Lys Gly Val Thr Ser Leu Lys Val Gly Asp Asn Val
            180                 185                 190

Ala Ile Glu Pro Gly Ile Pro Ser Arg Phe Ser Asp Glu Tyr Lys Ser
        195                 200                 205

Gly His Tyr Asn Leu Cys Pro His Met Ala Phe Ala Ala Thr Pro Asn
    210                 215                 220

Ser Lys Glu Gly Glu Pro Asn Pro Pro Gly Thr Leu Cys Lys Tyr Phe
225                 230                 235                 240

Lys Ser Pro Glu Asp Phe Leu Val Lys Leu Pro Asp His Val Ser Leu
                245                 250                 255

Glu Leu Gly Ala Leu Val Glu Pro Leu Ser Val Gly Val His Ala Ser
            260                 265                 270

Lys Leu Gly Ser Val Ala Phe Gly Asp Tyr Val Ala Val Phe Gly Ala
        275                 280                 285

Gly Pro Val Gly Leu Leu Ala Ala Ala Val Ala Lys Thr Phe Gly Ala
    290                 295                 300

Lys Gly Val Ile Val Val Asp Ile Phe Asp Asn Lys Leu Lys Met Ala
305                 310                 315                 320

Lys Asp Ile Gly Ala Ala Thr His Thr Phe Asn Ser Lys Thr Gly Gly
                325                 330                 335

Ser Glu Glu Leu Ile Lys Ala Phe Gly Gly Asn Val Pro Asn Val Val
            340                 345                 350

Leu Glu Cys Thr Gly Ala Glu Pro Cys Ile Lys Leu Gly Val Asp Ala
        355                 360                 365

Ile Ala Pro Gly Gly Arg Phe Val Gln Val Gly Asn Ala Ala Gly Pro
    370                 375                 380

Val Ser Phe Pro Ile Thr Val Phe Ala Met Lys Glu Leu Thr Leu Phe
385                 390                 395                 400

Gly Ser Phe Arg Tyr Gly Phe Asn Asp Tyr Lys Thr Ala Val Gly Ile
                405                 410                 415

Phe Asp Thr Asn Tyr Gln Asn Gly Arg Glu Asn Ala Pro Ile Asp Phe
            420                 425                 430

Glu Gln Leu Ile Thr His Arg Tyr Lys Phe Lys Asp Ala Ile Glu Ala
        435                 440                 445

Tyr Asp Leu Val Arg Ala Gly Lys Gly Ala Val Lys Cys Leu Ile Asp
    450                 455                 460

Gly Pro Glu Met Gln Ile Phe Val Lys Thr Leu Thr Gly Lys Thr Ile
465                 470                 475                 480

Thr Leu Glu Val Glu Pro Ser Asp Thr Ile Glu Asn Val Lys Ala Lys
                485                 490                 495

Ile Gln Asp Lys Glu Gly Ile Pro Pro Asp Gln Gln Arg Leu Ile Phe
            500                 505                 510

Ala Gly Lys Gln Leu Glu Asp Gly Arg Thr Leu Ser Asp Tyr Asn Ile
        515                 520                 525

Gln Lys Glu Ser Thr Leu His Leu Val Leu Arg Leu Arg Gly
    530                 535                 540

<210>    4
<211>    407
<212>    PRT
<213>    Artificial Sequence

<220>
<223>    Oxaloacetate decarboxylase

<400>    4
Met Gly His His His His His His Ser Asp Gln Glu Ala Lys Pro Ser
1               5                   10                  15

Thr Glu Asp Leu Gly Asp Lys Lys Glu Gly Glu Tyr Ile Lys Leu Lys
            20                  25                  30

Val Ile Gly Gln Asp Ser Ser Glu Ile His Phe Lys Val Lys Met Thr
        35                  40                  45

Thr His Leu Lys Lys Leu Lys Glu Ser Tyr Cys Gln Arg Gln Gly Val
    50                  55                  60

Pro Met Asn Ser Leu Arg Phe Leu Phe Glu Gly Gln Arg Ile Ala Asp
65                  70                  75                  80

Asn His Thr Pro Lys Glu Leu Gly Met Glu Glu Glu Asp Val Ile Glu
                85                  90                  95

Val Tyr Gln Glu Gln Thr Gly Gly Met Tyr Glu Leu Gly Val Val Tyr
            100                 105                 110

Arg Asn Ile Gln Arg Ala Asp Arg Ala Ala Ala Asp Gly Leu Ala Ala
        115                 120                 125

Leu Gly Ser Ala Thr Val His Glu Ala Met Gly Arg Val Gly Leu Leu
    130                 135                 140

Lys Pro Tyr Met Arg Pro Ile Tyr Ala Gly Lys Gln Val Ser Gly Thr
145                 150                 155                 160

Ala Val Thr Val Leu Leu Gln Pro Gly Asp Asn Trp Met Met His Val
                165                 170                 175

Ala Ala Glu Gln Ile Gln Pro Gly Asp Ile Val Val Ala Ala Val Thr
            180                 185                 190

Ala Glu Cys Thr Asp Gly Tyr Phe Gly Asp Leu Leu Ala Thr Ser Phe
        195                 200                 205

Gln Ala Arg Gly Ala Arg Ala Leu Ile Ile Asp Ala Gly Val Arg Asp
    210                 215                 220

Val Lys Thr Leu Gln Glu Met Asp Phe Pro Val Trp Ser Lys Ala Ile

225                 230                 235                 240

Ser Ser Lys Gly Thr Ile Lys Ala Thr Leu Gly Ser Val Asn Ile Pro
                245                 250                 255

Ile Val Cys Ala Gly Met Leu Val Thr Pro Gly Asp Val Ile Val Ala
            260                 265                 270

Asp Asp Asp Gly Val Val Cys Val Pro Ala Ala Arg Ala Val Glu Val
        275                 280                 285

Leu Ala Ala Ala Gln Lys Arg Glu Ser Phe Glu Gly Glu Lys Arg Ala
    290                 295                 300

Lys Leu Ala Ser Gly Val Leu Gly Leu Asp Met Tyr Lys Met Arg Glu
305                 310                 315                 320

Pro Leu Glu Lys Ala Gly Leu Lys Tyr Ile Asp Met Gln Ile Phe Val
                325                 330                 335

Lys Thr Leu Thr Gly Lys Thr Ile Thr Leu Glu Val Glu Pro Ser Asp
            340                 345                 350

Thr Ile Glu Asn Val Lys Ala Lys Ile Gln Asp Lys Glu Gly Ile Pro
        355                 360                 365

Pro Asp Gln Gln Arg Leu Ile Phe Ala Gly Lys Gln Leu Glu Asp Gly
    370                 375                 380

Arg Thr Leu Ser Asp Tyr Asn Ile Gln Lys Glu Ser Thr Leu His Leu
385                 390                 395                 400

Val Leu Arg Leu Arg Gly Gly
                405


<210>    5
<211>    430
<212>    PRT
<213>    Artificial Sequence

<220>
<223>    Triose-phosphate isomerase


<400>    5
Met Gly His His His His His His Ser Asp Gln Glu Ala Lys Pro Ser
1               5                   10                  15

Thr Glu Asp Leu Gly Asp Lys Lys Glu Gly Glu Tyr Ile Lys Leu Lys
            20                  25                  30

Val Ile Gly Gln Asp Ser Ser Glu Ile His Phe Lys Val Lys Met Thr
        35                  40                  45

Thr His Leu Lys Lys Leu Lys Glu Ser Tyr Cys Gln Arg Gln Gly Val
    50                  55                  60

Pro Met Asn Ser Leu Arg Phe Leu Phe Glu Gly Gln Arg Ile Ala Asp
65                  70                  75                  80

Asn His Thr Pro Lys Glu Leu Gly Met Glu Glu Glu Asp Val Ile Glu
                85                  90                  95

Val Tyr Gln Glu Gln Thr Gly Gly Met Arg Arg Val Leu Val Ala Gly
            100                 105                 110

Asn Trp Lys Met His Lys Thr Pro Ser Glu Ala Arg Val Trp Phe Ala
        115                 120                 125

Glu Leu Lys Arg Leu Leu Pro Pro Leu Gln Ser Glu Ala Ala Val Leu
    130                 135                 140

Pro Ala Phe Pro Ile Leu Pro Val Ala Lys Glu Val Leu Ala Glu Thr
145                 150                 155                 160

Gln Val Gly Tyr Gly Ala Gln Asp Val Ser Ala His Lys Glu Gly Ala
                165                 170                 175

Tyr Thr Gly Glu Val Ser Ala Arg Met Leu Ser Asp Leu Gly Cys Arg
            180                 185                 190

Tyr Ala Ile Val Gly His Ser Glu Arg Arg Arg Tyr His Gly Glu Thr
        195                 200                 205

Asp Ala Leu Val Ala Glu Lys Ala Lys Arg Leu Leu Glu Glu Gly Ile
    210                 215                 220

Thr Pro Ile Leu Cys Val Gly Glu Pro Leu Glu Val Arg Glu Lys Gly
225                 230                 235                 240

Glu Ala Val Pro Tyr Thr Leu Arg Gln Leu Arg Gly Ser Leu Glu Gly
                245                 250                 255

Val Glu Pro Pro Gly Pro Glu Ala Leu Val Ile Ala Tyr Glu Pro Val
            260                 265                 270

Trp Ala Ile Gly Thr Gly Lys Asn Ala Thr Pro Glu Asp Ala Glu Ala
        275                 280                 285

Met His Gln Glu Ile Arg Lys Ala Leu Ser Glu Arg Tyr Gly Glu Ala
    290                 295                 300

Phe Ala Ser Arg Val Arg Ile Leu Tyr Gly Gly Ser Val Asn Pro Lys
305                 310                 315                 320

Asn Phe Ala Asp Leu Leu Ser Met Pro Asn Val Asp Gly Gly Leu Val
                325                 330                 335

Gly Gly Ala Ser Leu Glu Leu Glu Ser Phe Leu Ala Leu Leu Arg Ile
            340                 345                 350

Ala Gly Met Gln Ile Phe Val Lys Thr Leu Thr Gly Lys Thr Ile Thr
        355                 360                 365

Leu Glu Val Glu Pro Ser Asp Thr Ile Glu Asn Val Lys Ala Lys Ile
    370                 375                 380

Gln Asp Lys Glu Gly Ile Pro Pro Asp Gln Gln Arg Leu Ile Phe Ala
385                 390                 395                 400

Gly Lys Gln Leu Glu Asp Gly Arg Thr Leu Ser Asp Tyr Asn Ile Gln
                405                 410                 415

Lys Glu Ser Thr Leu His Leu Val Leu Arg Leu Arg Gly Gly
            420                 425                 430

<210>   6
<211>   496
<212>   PRT
<213>   Artificial Sequence

<220>
<223>   Aldolase


<400>   6
Met Gly His His His His His His Ser Asp Gln Glu Ala Lys Pro Ser
1               5                   10                  15

Thr Glu Asp Leu Gly Asp Lys Lys Glu Gly Glu Tyr Ile Lys Leu Lys
            20                  25                  30

Val Ile Gly Gln Asp Ser Ser Glu Ile His Phe Lys Val Lys Met Thr
        35                  40                  45

Thr His Leu Lys Lys Leu Lys Glu Ser Tyr Cys Gln Arg Gln Gly Val
    50                  55                  60

Pro Met Asn Ser Leu Arg Phe Leu Phe Glu Gly Gln Arg Ile Ala Asp
65                  70                  75                  80

Asn His Thr Pro Lys Glu Leu Gly Met Glu Glu Glu Asp Val Ile Glu
                85                  90                  95

Val Tyr Gln Glu Gln Thr Gly Gly Met Val Thr Met Pro Tyr Val Lys
            100                 105                 110

Asn Thr Lys Glu Ile Leu Glu Lys Ala Ser Lys Glu Arg Tyr Ala Ile
        115                 120                 125

Gly Ala Phe Asn Phe Asn Asn Met Glu Phe Leu Gln Ala Ile Leu Glu
    130                 135                 140

Ala Ala Glu Glu Glu Lys Ala Pro Val Ile Val Ala Thr Ser Glu Gly
145                 150                 155                 160

Ala Ile Lys Tyr Ile Gly Lys Gly Asp Ile Glu Thr Gly Ala Lys Leu
                165                 170                 175

Ala Val Glu Met Val Arg Thr Tyr Ala Glu Lys Leu Ser Val Pro Val
            180                 185                 190

Ala Leu His Leu Asp His Gly Arg Asp Phe Lys Val Ile Met Ala Ala
        195                 200                 205

Ile Lys Ala Gly Tyr Ser Ser Val Met Ile Asp Ala Ser His Leu Pro
    210                 215                 220

Phe Glu Glu Asn Leu Arg Glu Thr Lys Arg Ile Val Glu Ile Ala His
225                 230                 235                 240

Ala Val Gly Ile Ser Val Glu Ala Glu Leu Gly Lys Leu Lys Gly Ile
                245                 250                 255

Glu Asp Asn Val Val Glu Lys Glu Ser Val Leu Val Asp Pro Glu Glu
            260                 265                 270

Ala Lys Val Phe Val Lys Glu Thr Glu Val Asp Phe Leu Ala Pro Ala
        275                 280                 285

Ile Gly Thr Ser His Gly Ala Phe Lys Phe Lys Gly Glu Ala Gln Leu
    290                 295                 300

Asp Phe Glu Arg Leu Lys Lys Val Lys Glu Tyr Thr Gln Ile Pro Leu
305                 310                 315                 320

Val Leu His Gly Ala Ser Met Val Pro Gln Asp Ile Val Lys Leu Ala
                325                 330                 335

Asn Glu Tyr Gly Ala Glu Leu Ser Gly Ala Lys Gly Val Pro Glu Asp
            340                 345                 350

Met Leu Lys Lys Ala Ile Glu Leu Gly Ile Asn Lys Ile Asn Thr Asp
        355                 360                 365

Thr Asp Leu Arg Ile Thr Phe Val Ala Tyr Leu Arg Lys Val Leu Ser
    370                 375                 380

Glu Asp Lys Ser Gln Ile Asp Pro Arg Lys Ile Phe Lys Pro Val Phe
385                 390                 395                 400

Glu Gln Val Lys Glu Ile Val Lys Glu Arg Ile Arg Ile Phe Gly Ser
                405                 410                 415

Ser Gly Lys Ala Met Gln Ile Phe Val Lys Thr Leu Thr Gly Lys Thr
            420                 425                 430

Ile Thr Leu Glu Val Glu Pro Ser Asp Thr Ile Glu Asn Val Lys Ala
        435                 440                 445

Lys Ile Gln Asp Lys Glu Gly Ile Pro Pro Asp Gln Gln Arg Leu Ile
    450                 455                 460

Phe Ala Gly Lys Gln Leu Glu Asp Gly Arg Thr Leu Ser Asp Tyr Asn
465                 470                 475                 480

Ile Gln Lys Glu Ser Thr Leu His Leu Val Leu Arg Leu Arg Gly Gly
                485                 490                 495



<210>   7
<211>   437
<212>   PRT
<213>   Artificial Sequence

<220>
<223>   Fructose 1,6-bisphosphatase


<400>   7
Met Gly His His His His His His Ser Asp Gln Glu Ala Lys Pro Ser
  1               5                  10                  15

Thr Glu Asp Leu Gly Asp Lys Lys Glu Gly Glu Tyr Ile Lys Leu Lys
             20                  25                  30

Val Ile Gly Gln Asp Ser Ser Glu Ile His Phe Lys Val Lys Met Thr
         35                  40                  45

Thr His Leu Lys Lys Leu Lys Glu Ser Tyr Cys Gln Arg Gln Gly Val
    50                  55                  60

Pro Met Asn Ser Leu Arg Phe Leu Phe Glu Gly Gln Arg Ile Ala Asp
65                  70                  75                  80

Asn His Thr Pro Lys Glu Leu Gly Met Glu Glu Glu Asp Val Ile Glu
                85                  90                  95

Val Tyr Gln Glu Gln Thr Gly Gly Met Leu Asp Arg Leu Asp Phe Ser
            100                 105                 110

Ile Lys Leu Leu Arg Lys Val Gly His Leu Leu Met Ile His Trp Gly
        115                 120                 125

Arg Val Asp Asn Val Glu Lys Lys Thr Gly Phe Lys Asp Ile Val Thr
    130                 135                 140

Glu Ile Asp Arg Glu Ala Gln Arg Met Ile Val Asp Glu Ile Arg Lys
145                 150                 155                 160

Phe Phe Pro Asp Glu Asn Ile Met Ala Glu Glu Gly Ile Phe Glu Lys
                165                 170                 175

Gly Asp Arg Leu Trp Ile Ile Asp Pro Ile Asp Gly Thr Ile Asn Phe
            180                 185                 190

Val His Gly Leu Pro Asn Phe Ser Ile Ser Leu Ala Tyr Val Glu Asn
        195                 200                 205

Gly Glu Val Lys Leu Gly Val Val His Ala Pro Ala Leu Asn Glu Thr
    210                 215                 220

Leu Tyr Ala Glu Glu Gly Ser Gly Ala Phe Phe Asn Gly Glu Arg Ile
225                 230                 235                 240

Arg Val Ser Glu Asn Ala Ser Leu Glu Glu Cys Val Gly Ser Thr Gly
                245                 250                 255

Ser Tyr Val Asp Phe Thr Gly Lys Phe Ile Glu Arg Met Glu Lys Arg
            260                 265                 270

Thr Arg Arg Ile Arg Ile Leu Gly Ser Ala Ala Leu Asn Ala Ala Tyr
        275                 280                 285

Val Gly Ala Gly Arg Val Asp Phe Phe Val Thr Trp Arg Ile Asn Pro
    290                 295                 300

Trp Asp Ile Ala Ala Gly Leu Ile Ile Val Lys Glu Ala Gly Gly Met
305                 310                 315                 320

Val Thr Asp Phe Ser Gly Lys Glu Ala Asn Ala Phe Ser Lys Asn Phe
                325                 330                 335

Ile Phe Ser Asn Gly Leu Ile His Asp Glu Val Val Lys Val Val Asn
            340                 345                 350

Glu Val Val Glu Glu Ile Gly Gly Lys Met Gln Ile Phe Val Lys Thr
        355                 360                 365

Leu Thr Gly Lys Thr Ile Thr Leu Glu Val Glu Pro Ser Asp Thr Ile
    370                 375                 380

Glu Asn Val Lys Ala Lys Ile Gln Asp Lys Glu Gly Ile Pro Pro Asp
385                 390                 395                 400

Gln Gln Arg Leu Ile Phe Ala Gly Lys Gln Leu Glu Asp Gly Arg Thr
                405                 410                 415

Leu Ser Asp Tyr Asn Ile Gln Lys Glu Ser Thr Leu His Leu Val Leu
            420                 425                 430

Arg Leu Arg Gly Gly
        435

<210>   8
<211>   772
<212>   PRT
<213>   Artificial Sequence

<220>
<223>   Pyruvate oxidase

<400>   8
Met Gly His His His His His His Ser Asp Gln Glu Ala Lys Pro Ser
  1               5                  10                  15

Thr Glu Asp Leu Gly Asp Lys Lys Glu Gly Glu Tyr Ile Lys Leu Lys
             20                  25                  30

Val Ile Gly Gln Asp Ser Ser Glu Ile His Phe Lys Val Lys Met Thr
         35                  40                  45

Thr His Leu Lys Lys Leu Lys Glu Ser Tyr Cys Gln Arg Gln Gly Val
     50                  55                  60

Pro Met Asn Ser Leu Arg Phe Leu Phe Glu Gly Gln Arg Ile Ala Asp
 65                  70                  75                  80

Asn His Thr Pro Lys Glu Leu Gly Met Glu Glu Glu Asp Val Ile Glu
                 85                  90                  95

Val Tyr Gln Glu Gln Thr Gly Gly Met Ser Asp Asn Lys Ile Asn Ile
            100                 105                 110

Gly Leu Ala Val Met Lys Ile Leu Glu Ser Trp Gly Ala Asp Thr Ile
        115                 120                 125

Tyr Gly Ile Pro Ser Gly Thr Leu Ser Ser Leu Met Asp Ala Met Gly
    130                 135                 140

Glu Glu Glu Asn Asn Val Lys Phe Leu Gln Val Lys His Glu Glu Val
145                 150                 155                 160

Gly Ala Met Ala Ala Val Met Gln Ser Lys Phe Gly Gly Asn Leu Gly
                165                 170                 175

Val Thr Val Gly Ser Gly Gly Pro Gly Ala Ser His Leu Ile Asn Gly
            180                 185                 190

Leu Tyr Asp Ala Ala Met Asp Asn Ile Pro Val Val Ala Ile Leu Gly
        195                 200                 205

Ser Arg Pro Gln Arg Glu Leu Asn Met Asp Ala Phe Gln Glu Leu Asn

210                 215                 220

Gln Asn Pro Met Tyr Asp His Ile Ala Val Tyr Asn Arg Arg Val Ala
225                 230                 235                 240

Tyr Ala Glu Gln Leu Pro Lys Leu Val Asp Glu Ala Ala Arg Met Ala
                245                 250                 255

Ile Ala Lys Arg Gly Val Ala Val Leu Glu Val Pro Gly Asp Phe Ala
            260                 265                 270

Lys Val Glu Ile Asp Asn Asp Gln Trp Tyr Ser Ser Ala Asn Ser Leu
        275                 280                 285

Arg Lys Tyr Ala Pro Ile Ala Pro Ala Ala Gln Asp Ile Asp Ala Ala
    290                 295                 300

Val Glu Leu Leu Asn Asn Ser Lys Arg Pro Val Ile Tyr Ala Gly Ile
305                 310                 315                 320

Gly Thr Met Gly His Gly Pro Ala Val Gln Glu Leu Ala Arg Lys Ile
                325                 330                 335

Lys Ala Pro Val Ile Thr Thr Gly Lys Asn Phe Glu Thr Phe Glu Trp
            340                 345                 350

Asp Phe Glu Ala Leu Thr Gly Ser Thr Tyr Arg Val Gly Trp Lys Pro
        355                 360                 365

Ala Asn Glu Thr Ile Leu Glu Ala Asp Thr Val Leu Phe Ala Gly Ser
    370                 375                 380

Asn Phe Pro Phe Ser Glu Val Glu Gly Thr Phe Arg Asn Val Asp Asn
385                 390                 395                 400

Phe Ile Gln Ile Asp Ile Asp Pro Ala Met Leu Gly Lys Arg His His
                405                 410                 415

Ala Asp Val Ala Ile Leu Gly Asp Ala Gly Leu Ala Ile Asp Glu Ile
            420                 425                 430

Leu Asn Lys Val Asp Ala Val Glu Glu Ser Ala Trp Trp Thr Ala Asn
        435                 440                 445

Leu Lys Asn Ile Ala Asn Trp Arg Glu Tyr Ile Asn Met Leu Glu Thr
    450                 455                 460

Lys Glu Glu Gly Asp Leu Gln Phe Tyr Gln Val Tyr Asn Ala Ile Asn
465                 470                 475                 480

Asn His Ala Asp Glu Asp Ala Ile Tyr Ser Ile Asp Val Gly Asn Ser
                485                 490                 495

Thr Gln Thr Ser Ile Arg His Leu His Met Thr Pro Lys Asn Met Trp
            500                 505                 510

Arg Thr Ser Pro Leu Phe Ala Thr Met Gly Ile Ala Ile Pro Gly Gly
        515                 520                 525

Leu Gly Ala Lys Asn Thr Tyr Pro Asp Arg Gln Val Trp Asn Ile Ile
    530                 535                 540

Gly Asp Gly Ala Phe Ser Met Thr Tyr Pro Asp Val Val Thr Asn Val

545                 550                 555                 560

Arg Tyr Asn Met Pro Val Ile Asn Val Val Phe Ser Asn Thr Glu Tyr
                565                 570                 575

Ala Phe Ile Lys Asn Lys Tyr Glu Asp Thr Asn Lys Asn Leu Phe Gly
            580                 585                 590

Val Asp Phe Thr Asp Val Asp Tyr Ala Lys Ile Ala Glu Ala Gln Gly
        595                 600                 605

Ala Lys Gly Phe Thr Val Ser Arg Ile Glu Asp Met Asp Arg Val Met
    610                 615                 620

Ala Glu Ala Val Ala Ala Asn Lys Ala Gly His Thr Val Val Ile Asp
625                 630                 635                 640

Cys Lys Ile Thr Gln Asp Arg Pro Ile Pro Val Glu Thr Leu Lys Leu
                645                 650                 655

Asp Ser Lys Leu Tyr Ser Glu Asp Glu Ile Lys Ala Tyr Lys Glu Arg
            660                 665                 670

Tyr Glu Ala Ala Asn Leu Val Pro Phe Arg Glu Tyr Leu Glu Ala Glu
        675                 680                 685

Gly Leu Glu Ser Lys Tyr Ile Lys Met Gln Ile Phe Val Lys Thr Leu
    690                 695                 700

Thr Gly Lys Thr Ile Thr Leu Glu Val Glu Pro Ser Asp Thr Ile Glu
705                 710                 715                 720

Asn Val Lys Ala Lys Ile Gln Asp Lys Glu Gly Ile Pro Pro Asp Gln
                725                 730                 735

Gln Arg Leu Ile Phe Ala Gly Lys Gln Leu Glu Asp Gly Arg Thr Leu
            740                 745                 750

Ser Asp Tyr Asn Ile Gln Lys Glu Ser Thr Leu His Leu Val Leu Arg
        755                 760                 765

Leu Arg Gly Gly
    770


<210>    9
<211>    1024
<212>    PRT
<213>    Artificial Sequence

<220>
<223>    Yeast UBE1


<400>    9
Met Ser Ser Asn Asn Ser Gly Leu Ser Ala Ala Gly Glu Ile Asp Glu
1               5                   10                  15

Ser Leu Tyr Ser Arg Gln Leu Tyr Val Leu Gly Lys Glu Ala Met Leu
            20                  25                  30

Lys Met Gln Thr Ser Asn Val Leu Ile Leu Gly Leu Lys Gly Leu Gly
        35                  40                  45

Val Glu Ile Ala Lys Asn Val Val Leu Ala Gly Val Lys Ser Met Thr
    50                  55                  60

Val Phe Asp Pro Glu Pro Val Gln Leu Ala Asp Leu Ser Thr Gln Phe
65                  70                  75                  80

Phe Leu Thr Glu Lys Asp Ile Gly Gln Lys Arg Gly Asp Val Thr Arg
                85                  90                  95

Ala Lys Leu Ala Glu Leu Asn Ala Tyr Val Pro Val Asn Val Leu Asp
            100                 105                 110

Ser Leu Asp Asp Val Thr Gln Leu Ser Gln Phe Gln Val Val Val Ala
        115                 120                 125

Thr Asp Thr Val Ser Leu Glu Asp Lys Val Lys Ile Asn Glu Phe Cys
    130                 135                 140

His Ser Ser Gly Ile Arg Phe Ile Ser Ser Glu Thr Arg Gly Leu Phe
145                 150                 155                 160

Gly Asn Thr Phe Val Asp Leu Gly Asp Glu Phe Thr Val Leu Asp Pro
                165                 170                 175

Thr Gly Glu Glu Pro Arg Thr Gly Met Val Ser Asp Ile Glu Pro Asp
            180                 185                 190

Gly Thr Val Thr Met Leu Asp Asp Asn Arg His Gly Leu Glu Asp Gly
        195                 200                 205

Asn Phe Val Arg Phe Ser Glu Val Glu Gly Leu Asp Lys Leu Asn Asp
    210                 215                 220

Gly Thr Leu Phe Lys Val Glu Val Leu Gly Pro Phe Ala Phe Arg Ile
225                 230                 235                 240

Gly Ser Val Lys Glu Tyr Gly Glu Tyr Lys Lys Gly Gly Ile Phe Thr
                245                 250                 255

Glu Val Lys Val Pro Arg Lys Ile Ser Phe Lys Ser Leu Lys Gln Gln
            260                 265                 270

Leu Ser Asn Pro Glu Phe Val Phe Ser Asp Phe Ala Lys Phe Asp Arg
        275                 280                 285

Ala Ala Gln Leu His Leu Gly Phe Gln Ala Leu His Gln Phe Ala Val
    290                 295                 300

Arg His Asn Gly Glu Leu Pro Arg Thr Met Asn Asp Glu Asp Ala Asn
305                 310                 315                 320

Glu Leu Ile Lys Leu Val Thr Asp Leu Ser Val Gln Gln Pro Glu Val
                325                 330                 335

Leu Gly Glu Gly Val Asp Val Asn Glu Asp Leu Ile Lys Glu Leu Ser
            340                 345                 350

Tyr Gln Ala Arg Gly Asp Ile Pro Gly Val Val Ala Phe Phe Gly Gly
        355                 360                 365

Leu Val Ala Gln Glu Val Leu Lys Ala Cys Ser Gly Lys Phe Thr Pro
    370                 375                 380

Leu Lys Gln Phe Met Tyr Phe Asp Ser Leu Glu Ser Leu Pro Asp Pro
385                 390                 395                 400

Lys Asn Phe Pro Arg Asn Glu Lys Thr Thr Gln Pro Val Asn Ser Arg
                405                 410                 415

Tyr Asp Asn Gln Ile Ala Val Phe Gly Leu Asp Phe Gln Lys Lys Ile
            420                 425                 430

Ala Asn Ser Lys Val Phe Leu Val Gly Ser Gly Ala Ile Gly Cys Glu
        435                 440                 445

Met Leu Lys Asn Trp Ala Leu Leu Gly Leu Gly Ser Gly Ser Asp Gly
    450                 455                 460

Tyr Ile Val Val Thr Asp Asn Asp Ser Ile Glu Lys Ser Asn Leu Asn
465                 470                 475                 480

Arg Gln Phe Leu Phe Arg Pro Lys Asp Val Gly Lys Asn Lys Ser Glu
                485                 490                 495

Val Ala Ala Glu Ala Val Cys Ala Met Asn Pro Asp Leu Lys Gly Lys
            500                 505                 510

Ile Asn Ala Lys Ile Asp Lys Val Gly Pro Glu Thr Glu Glu Ile Phe
        515                 520                 525

Asn Asp Ser Phe Trp Glu Ser Leu Asp Phe Val Thr Asn Ala Leu Asp
    530                 535                 540

Asn Val Asp Ala Arg Thr Tyr Val Asp Arg Arg Cys Val Phe Tyr Arg
545                 550                 555                 560

Lys Pro Leu Leu Glu Ser Gly Thr Leu Gly Thr Lys Gly Asn Thr Gln
                565                 570                 575

Val Ile Ile Pro Arg Leu Thr Glu Ser Tyr Ser Ser Ser Arg Asp Pro
            580                 585                 590

Pro Glu Lys Ser Ile Pro Leu Cys Thr Leu Arg Ser Phe Pro Asn Lys
        595                 600                 605

Ile Asp His Thr Ile Ala Trp Ala Lys Ser Leu Phe Gln Gly Tyr Phe
    610                 615                 620

Thr Asp Ser Ala Glu Asn Val Asn Met Tyr Leu Thr Gln Pro Asn Phe
625                 630                 635                 640

Val Glu Gln Thr Leu Lys Gln Ser Gly Asp Val Lys Gly Val Leu Glu
                645                 650                 655

Ser Ile Ser Asp Ser Leu Ser Ser Lys Pro His Asn Phe Glu Asp Cys
            660                 665                 670

Ile Lys Trp Ala Arg Leu Glu Phe Glu Lys Lys Phe Asn His Asp Ile
        675                 680                 685

Lys Gln Leu Leu Phe Asn Phe Pro Lys Asp Ala Lys Thr Ser Asn Gly
    690                 695                 700

Glu Pro Phe Trp Ser Gly Ala Lys Arg Ala Pro Thr Pro Leu Glu Phe
705                 710                 715                 720

Asp Ile Tyr Asn Asn Asp His Phe His Phe Val Val Ala Gly Ala Ser
                725                 730                 735

Leu Arg Ala Tyr Asn Tyr Gly Ile Lys Ser Asp Asp Ser Asn Ser Lys
            740                 745                 750

Pro Asn Val Asp Glu Tyr Lys Ser Val Ile Asp His Met Ile Ile Pro
        755                 760                 765

Glu Phe Thr Pro Asn Ala Asn Leu Lys Ile Gln Val Asn Asp Asp Asp
    770                 775                 780

Pro Asp Pro Asn Ala Asn Ala Ala Asn Gly Ser Asp Glu Ile Asp Gln
785                 790                 795                 800

Leu Val Ser Ser Leu Pro Asp Pro Ser Thr Leu Ala Gly Phe Lys Leu
                805                 810                 815

Glu Pro Val Asp Phe Glu Lys Asp Asp Asp Thr Asn His His Ile Glu
            820                 825                 830

Phe Ile Thr Ala Cys Ser Asn Cys Arg Ala Gln Asn Tyr Phe Ile Glu
        835                 840                 845

Thr Ala Asp Arg Gln Lys Thr Lys Phe Ile Ala Gly Arg Ile Ile Pro
    850                 855                 860

Ala Ile Ala Thr Thr Thr Ser Leu Val Thr Gly Leu Val Asn Leu Glu
865                 870                 875                 880

Leu Tyr Lys Leu Ile Asp Asn Lys Thr Asp Ile Glu Gln Tyr Lys Asn
                885                 890                 895

Gly Phe Val Asn Leu Ala Leu Pro Phe Phe Gly Phe Ser Glu Pro Ile
            900                 905                 910

Ala Ser Pro Lys Gly Glu Tyr Asn Asn Lys Lys Tyr Asp Lys Ile Trp
        915                 920                 925

Asp Arg Phe Asp Ile Lys Gly Asp Ile Lys Leu Ser Asp Leu Ile Glu
    930                 935                 940

His Phe Glu Lys Asp Glu Gly Leu Glu Ile Thr Met Leu Ser Tyr Gly
945                 950                 955                 960

Val Ser Leu Leu Tyr Ala Ser Phe Phe Pro Pro Lys Lys Leu Lys Glu
                965                 970                 975

Arg Leu Asn Leu Pro Ile Thr Gln Leu Val Lys Leu Val Thr Lys Lys
            980                 985                 990

Asp Ile Pro Ala His Val Ser Thr Met Ile Leu Glu Ile Cys Ala Asp
        995                 1000                1005

Asp Lys Glu Gly Glu Asp Val Glu Val Pro Phe Ile Thr Ile His Leu
    1010                1015                1020

<210>   10
<211>   147

<212>    PRT
<213>    Artificial Sequence

<220>
<223>    Ubch5a (Homo sapiens)


<400>    10
Met Ala Leu Lys Arg Ile Gln Lys Glu Leu Ser Asp Leu Gln Arg Asp
1               5                   10                  15

Pro Pro Ala His Cys Ser Ala Gly Pro Val Gly Asp Asp Leu Phe His
            20                  25                  30

Trp Gln Ala Thr Ile Met Gly Pro Pro Asp Ser Ala Tyr Gln Gly Gly
        35                  40                  45

Val Phe Phe Leu Thr Val His Phe Pro Thr Asp Tyr Pro Phe Lys Pro
    50                  55                  60

Pro Lys Ile Ala Phe Thr Thr Lys Ile Tyr His Pro Asn Ile Asn Ser
65                  70                  75                  80

Asn Gly Ser Ile Cys Leu Asp Ile Leu Arg Ser Gln Trp Ser Pro Ala
                85                  90                  95

Leu Thr Val Ser Lys Val Leu Leu Ser Ile Cys Ser Leu Leu Cys Asp
            100                 105                 110

Pro Asn Pro Asp Asp Pro Leu Val Pro Asp Ile Ala Gln Ile Tyr Lys
        115                 120                 125

Ser Asp Lys Glu Lys Tyr Asn Arg His Ala Arg Glu Trp Thr Gln Lys
    130                 135                 140

Tyr Ala Met
145


<210>    11
<211>    154
<212>    PRT
<213>    Artificial Sequence

<220>
<223>    Ubch7 (Homo sapiens)


<400>    11


<210>    12
<211>    200
<212>    PRT
<213>    Artificial Sequence

<220>
<223>    E2-25K (Homo sapiens)


<400>    12
Met Ala Asn Ile Ala Val Gln Arg Ile Lys Arg Glu Phe Lys Glu Val
 1               5                  10                  15

Leu Lys Ser Glu Glu Thr Ser Lys Asn Gln Ile Lys Val Asp Leu Val
            20                  25                  30

Asp Glu Asn Phe Thr Glu Leu Arg Gly Glu Ile Ala Gly Pro Pro Asp
        35                  40                  45

Thr Pro Tyr Glu Gly Gly Arg Tyr Gln Leu Glu Ile Lys Ile Pro Glu
    50                  55                  60

Thr Tyr Pro Phe Asn Pro Pro Lys Val Arg Phe Ile Thr Lys Ile Trp
65                  70                  75                  80

His Pro Asn Ile Ser Ser Val Thr Gly Ala Ile Cys Leu Asp Ile Leu
                85                  90                  95

Lys Asp Gln Trp Ala Ala Ala Met Thr Leu Arg Thr Val Leu Leu Ser
            100                 105                 110

Leu Gln Ala Leu Leu Ala Ala Ala Glu Pro Asp Asp Pro Gln Asp Ala
        115                 120                 125

Val Val Ala Asn Gln Tyr Lys Gln Asn Pro Glu Met Phe Lys Gln Thr
    130                 135                 140

Ala Arg Leu Trp Ala His Val Tyr Ala Gly Ala Pro Val Ser Ser Pro
145                 150                 155                 160

Glu Tyr Thr Lys Lys Ile Glu Asn Leu Cys Ala Met Gly Phe Asp Arg
                165                 170                 175

Asn Ala Val Ile Val Ala Leu Ser Ser Lys Ser Trp Asp Val Glu Thr
            180                 185                 190

Ala Thr Glu Leu Leu Leu Ser Asn
        195                 200

<210>    13
<211>    153
<212>    PRT
<213>    Artificial Sequence

<220>
<223>    Ubc13 (Saccharomyces cerevisiae)

<400>    13
Met Ala Ser Leu Pro Lys Arg Ile Ile Lys Glu Thr Glu Lys Leu Val
1               5                   10                  15

Ser Asp Pro Val Pro Gly Ile Thr Ala Glu Pro His Asp Asp Asn Leu
            20                  25                  30

Arg Tyr Phe Gln Val Thr Ile Glu Gly Pro Glu Gln Ser Pro Tyr Glu
        35                  40                  45

Asp Gly Ile Phe Glu Leu Glu Leu Tyr Leu Pro Asp Asp Tyr Pro Met
    50                  55                  60

Glu Ala Pro Lys Val Arg Phe Leu Thr Lys Ile Tyr His Pro Asn Ile
65                  70                  75                  80

Asp Arg Leu Gly Arg Ile Cys Leu Asp Val Leu Lys Thr Asn Trp Ser
                85                  90                  95

Pro Ala Leu Gln Ile Arg Thr Val Leu Leu Ser Ile Gln Ala Leu Leu
            100                 105                 110

Ala Ser Pro Asn Pro Asn Asp Pro Leu Ala Asn Asp Val Ala Glu Asp
        115                 120                 125

Trp Ile Lys Asn Glu Gln Gly Ala Lys Ala Lys Ala Arg Glu Trp Thr
    130                 135                 140

Lys Leu Tyr Ala Lys Lys Lys Pro Glu
145                 150

<210>    14
<211>    137
<212>    PRT
<213>    Artificial Sequence

<220>
<223>    MMS2 (UEV - Ubiquitin-conjugating enzyme variant) (Saccharomyces
         cerevisiae)

<400>    14
Met Ser Lys Val Pro Arg Asn Phe Arg Leu Leu Glu Glu Leu Glu Lys
1               5                   10                  15

Gly Glu Lys Gly Phe Gly Pro Glu Ser Cys Ser Tyr Gly Leu Ala Asp
            20                  25                  30

Ser Asp Asp Ile Thr Met Thr Lys Trp Asn Gly Thr Ile Leu Gly Pro

        35                  40                  45

Pro His Ser Asn His Glu Asn Arg Ile Tyr Ser Leu Ser Ile Asp Cys
    50                  55                  60

Gly Pro Asn Tyr Pro Asp Ser Pro Pro Lys Val Thr Phe Ile Ser Lys
65                  70                  75                  80

Ile Asn Leu Pro Cys Val Asn Pro Thr Thr Gly Glu Val Gln Thr Asp
                85                  90                  95

Phe His Thr Leu Arg Asp Trp Lys Arg Ala Tyr Thr Met Glu Thr Leu
            100                 105                 110

Leu Leu Asp Leu Arg Lys Glu Met Ala Thr Pro Ala Asn Lys Lys Leu
        115                 120                 125

Arg Gln Pro Lys Glu Gly Glu Thr Phe
    130                 135

<210> 15
<211> 807
<212> PRT
<213> Artificial Sequence

<220>
<223> E3 RSP5

<400> 15
Met Pro Ser Ser Ile Ser Val Lys Leu Val Ala Ala Glu Ser Leu Tyr
1               5                   10                  15

Lys Arg Asp Val Phe Arg Ser Pro Asp Pro Phe Ala Val Leu Thr Ile
            20                  25                  30

Asp Gly Tyr Gln Thr Lys Ser Thr Ser Ala Ala Lys Lys Thr Leu Asn
        35                  40                  45

Pro Tyr Trp Asn Glu Thr Phe Lys Phe Asp Asp Ile Asn Glu Asn Ser
    50                  55                  60

Ile Leu Thr Ile Gln Val Phe Asp Gln Lys Lys Phe Lys Lys Lys Asp
65                  70                  75                  80

Gln Gly Phe Leu Gly Val Val Asn Val Arg Val Gly Asp Val Leu Gly
                85                  90                  95

His Leu Asp Glu Asp Thr Ala Thr Ser Ser Gly Arg Pro Arg Glu Glu
            100                 105                 110

Thr Ile Thr Arg Asp Leu Lys Lys Ser Asn Asp Gly Met Ala Val Ser
        115                 120                 125

Gly Arg Leu Ile Val Val Leu Ser Lys Leu Pro Ser Ser Ser Pro His
    130                 135                 140

Ser Gln Ala Pro Ser Gly His Thr Ala Ser Ser Ser Thr Asn Thr Ser
145                 150                 155                 160

Ser Thr Thr Arg Thr Asn Gly His Ser Thr Ser Ser Thr Arg Asn His
                165                 170                 175

Ser Thr Ser His Pro Ser Arg Gly Thr Ala Gln Ala Val Glu Ser Thr
            180                 185                 190

Leu Gln Ser Gly Thr Thr Ala Ala Thr Asn Thr Ala Thr Thr Ser His
        195                 200                 205

Arg Ser Thr Asn Ser Thr Ser Ser Ala Thr Arg Gln Tyr Ser Ser Phe
    210                 215                 220

Glu Asp Gln Tyr Gly Arg Leu Pro Pro Gly Trp Glu Arg Arg Thr Asp
225                 230                 235                 240

Asn Phe Gly Arg Thr Tyr Tyr Val Asp His Asn Thr Arg Thr Thr Thr
                245                 250                 255

Trp Lys Arg Pro Thr Leu Asp Gln Thr Glu Ala Glu Arg Gly Gln Leu
            260                 265                 270

Asn Ala Asn Thr Glu Leu Glu Arg Arg Gln His Arg Gly Arg Thr Leu
        275                 280                 285

Pro Gly Gly Ser Ser Asp Asn Ser Ser Val Thr Val Gln Val Gly Gly
    290                 295                 300

Gly Ser Asn Ile Pro Pro Val Asn Gly Ala Ala Ala Ala Ala Phe Ala
305                 310                 315                 320

Ala Thr Gly Gly Thr Thr Ser Gly Leu Gly Glu Leu Pro Ser Gly Trp
                325                 330                 335

Glu Gln Arg Phe Thr Pro Glu Gly Arg Ala Tyr Phe Val Asp His Asn
            340                 345                 350

Thr Arg Thr Thr Thr Trp Val Asp Pro Arg Arg Gln Gln Tyr Ile Arg
        355                 360                 365

Thr Tyr Gly Pro Thr Asn Thr Thr Ile Gln Gln Gln Pro Val Ser Gln
    370                 375                 380

Leu Gly Pro Leu Pro Ser Gly Trp Glu Met Arg Leu Thr Asn Thr Ala
385                 390                 395                 400

Arg Val Tyr Phe Val Asp His Asn Thr Lys Thr Thr Thr Trp Asp Asp
                405                 410                 415

Pro Arg Leu Pro Ser Ser Leu Asp Gln Asn Val Pro Gln Tyr Lys Arg
            420                 425                 430

Asp Phe Arg Arg Lys Val Ile Tyr Phe Arg Ser Gln Pro Ala Leu Arg
        435                 440                 445

Ile Leu Pro Gly Gln Cys His Ile Lys Val Arg Arg Lys Asn Ile Phe
    450                 455                 460

Glu Asp Ala Tyr Gln Glu Ile Met Arg Gln Thr Pro Glu Asp Leu Lys
465                 470                 475                 480

Lys Arg Leu Met Ile Lys Phe Asp Gly Glu Glu Gly Leu Asp Tyr Gly
                485                 490                 495

Gly Val Ser Arg Glu Phe Phe Phe Leu Leu Ser His Glu Met Phe Asn
            500                 505                 510

Pro Phe Tyr Cys Leu Phe Glu Tyr Ser Ala Tyr Asp Asn Tyr Thr Ile
        515                 520                 525

Gln Ile Asn Pro Asn Ser Gly Ile Asn Pro Glu His Leu Asn Tyr Phe
    530                 535                 540

Lys Phe Ile Gly Arg Val Val Gly Leu Gly Val Phe His Arg Arg Phe
545                 550                 555                 560

Leu Asp Ala Phe Phe Val Gly Ala Leu Tyr Lys Met Met Leu Arg Lys
                565                 570                 575

Lys Val Val Leu Gln Asp Met Glu Gly Val Asp Ala Glu Val Tyr Asn
            580                 585                 590

Ser Leu Asn Trp Met Leu Glu Asn Ser Ile Asp Gly Val Leu Asp Leu
        595                 600                 605

Thr Phe Ser Ala Asp Asp Glu Arg Phe Gly Glu Val Val Thr Val Asp
    610                 615                 620

Leu Lys Pro Asp Gly Arg Asn Ile Glu Val Thr Asp Gly Asn Lys Lys
625                 630                 635                 640

Glu Tyr Val Glu Leu Tyr Thr Gln Trp Arg Ile Val Asp Arg Val Gln
                645                 650                 655

Glu Gln Phe Lys Ala Phe Met Asp Gly Phe Asn Glu Ile Pro Glu Asp
            660                 665                 670

Leu Val Thr Val Phe Asp Glu Arg Glu Leu Glu Leu Leu Ile Gly Gly
        675                 680                 685

Ile Ala Glu Ile Asp Ile Glu Asp Trp Lys Lys His Thr Asp Tyr Arg
    690                 695                 700

Gly Tyr Gln Glu Ser Asp Glu Val Ile Gln Trp Phe Trp Lys Cys Val
705                 710                 715                 720

Ser Glu Trp Asp Asn Glu Gln Arg Ala Arg Leu Leu Gln Phe Thr Thr
                725                 730                 735

Gly Thr Ser Arg Ile Pro Val Asn Gly Phe Lys Asp Leu Gln Gly Ser
            740                 745                 750

Asp Gly Pro Arg Arg Phe Thr Ile Glu Lys Ala Gly Glu Val Gln Gln
        755                 760                 765

Leu Pro Lys Ser His Thr Cys Phe Asn Arg Val Asp Leu Pro Gln Tyr
    770                 775                 780

Val Asp Tyr Asp Ser Met Lys Gln Lys Leu Thr Leu Ala Val Glu Glu
785                 790                 795                 800

Thr Ile Gly Phe Gly Gln Glu
                805

<210>  16
<211>  1319
<212>  PRT
<213>  Artificial Sequence

<220>
<223>    E3 DOA10


<400>    16
Met Asp Val Asp Ser Asp Val Asn Val Ser Arg Leu Arg Asp Glu Leu
1               5                   10                  15

His Lys Val Ala Asn Glu Glu Thr Asp Thr Ala Thr Phe Asn Asp Asp
            20                  25                  30

Ala Pro Ser Gly Ala Thr Cys Arg Ile Cys Arg Gly Glu Ala Thr Glu
        35                  40                  45

Asp Asn Pro Leu Phe His Pro Cys Lys Cys Arg Gly Ser Ile Lys Tyr
    50                  55                  60

Met His Glu Ser Cys Leu Leu Glu Trp Val Ala Ser Lys Asn Ile Asp
65                  70                  75                  80

Ile Ser Lys Pro Gly Ala Asp Val Lys Cys Asp Ile Cys His Tyr Pro
                85                  90                  95

Ile Gln Phe Lys Thr Ile Tyr Ala Glu Asn Met Pro Glu Lys Ile Pro
            100                 105                 110

Phe Ser Leu Leu Leu Ser Lys Ser Ile Leu Thr Phe Phe Glu Lys Ala
        115                 120                 125

Arg Leu Ala Leu Thr Ile Gly Leu Ala Ala Val Leu Tyr Ile Ile Gly
    130                 135                 140

Val Pro Leu Val Trp Asn Met Phe Gly Lys Leu Tyr Thr Met Met Leu
145                 150                 155                 160

Asp Gly Ser Ser Pro Tyr Pro Gly Asp Phe Leu Lys Ser Leu Ile Tyr
                165                 170                 175

Gly Tyr Asp Gln Ser Ala Thr Pro Glu Leu Thr Thr Arg Ala Ile Phe
            180                 185                 190

Tyr Gln Leu Leu Gln Asn His Ser Phe Thr Ser Leu Gln Phe Ile Met
        195                 200                 205

Ile Val Ile Leu His Ile Ala Leu Tyr Phe Gln Tyr Asp Met Ile Val
    210                 215                 220

Arg Glu Asp Val Phe Ser Lys Met Val Phe His Lys Ile Gly Pro Arg
225                 230                 235                 240

Leu Ser Pro Lys Asp Leu Lys Ser Arg Leu Lys Glu Arg Phe Pro Met
                245                 250                 255

Met Asp Asp Arg Met Val Glu Tyr Leu Ala Arg Glu Met Arg Ala His
            260                 265                 270

Asp Glu Asn Arg Gln Glu Gln Gly His Asp Arg Leu Asn Met Pro Ala
        275                 280                 285

Ala Ala Ala Asp Asn Asn Asn Asn Val Ile Asn Pro Arg Asn Asp Asn
    290                 295                 300

Val Pro Pro Gln Asp Pro Asn Asp His Arg Asn Phe Glu Asn Leu Arg
305                 310                 315                 320

His Val Asp Glu Leu Asp His Asp Glu Ala Thr Glu Glu His Glu Asn
                325                 330                 335

Asn Asp Ser Asp Asn Ser Leu Pro Ser Gly Asp Asp Ser Ser Arg Ile
            340                 345                 350

Leu Pro Gly Ser Ser Ser Asp Asn Glu Glu Asp Glu Glu Ala Glu Gly
        355                 360                 365

Gln Gln Gln Gln Gln Gln Pro Glu Glu Glu Ala Asp Tyr Arg Asp His
    370                 375                 380

Ile Glu Pro Asn Pro Ile Asp Met Trp Ala Asn Arg Arg Ala Gln Asn
385                 390                 395                 400

Glu Phe Asp Asp Leu Ile Ala Ala Gln Gln Asn Ala Ile Asn Arg Pro
                405                 410                 415

Asn Ala Pro Val Phe Ile Pro Pro Pro Ala Gln Asn Arg Ala Gly Asn
            420                 425                 430

Val Asp Gln Asp Glu Gln Asp Phe Gly Ala Ala Val Gly Val Pro Pro
        435                 440                 445

Ala Gln Ala Asn Pro Asp Asp Gln Gly Gln Gly Pro Leu Val Ile Asn
    450                 455                 460

Leu Lys Leu Lys Leu Leu Asn Val Ile Ala Tyr Phe Ile Ile Ala Val
465                 470                 475                 480

Val Phe Thr Ala Ile Tyr Leu Ala Ile Ser Tyr Leu Phe Pro Thr Phe
                485                 490                 495

Ile Gly Phe Gly Leu Leu Lys Ile Tyr Phe Gly Ile Phe Lys Val Ile
            500                 505                 510

Leu Arg Gly Leu Cys His Leu Tyr Tyr Leu Ser Gly Ala His Ile Ala
        515                 520                 525

Tyr Asn Gly Leu Thr Lys Leu Val Pro Lys Val Asp Val Ala Met Ser
    530                 535                 540

Trp Ile Ser Asp His Leu Ile His Asp Ile Ile Tyr Leu Tyr Asn Gly
545                 550                 555                 560

Tyr Thr Glu Asn Thr Met Lys His Ser Ile Phe Ile Arg Ala Leu Pro
                565                 570                 575

Ala Leu Thr Thr Tyr Leu Thr Ser Val Ser Ile Val Cys Ala Ser Ser
            580                 585                 590

Asn Leu Val Ser Arg Gly Tyr Gly Arg Glu Asn Gly Met Ser Asn Pro
        595                 600                 605

Thr Arg Arg Leu Ile Phe Gln Ile Leu Phe Ala Leu Lys Cys Thr Phe
    610                 615                 620

Lys Val Phe Thr Leu Phe Phe Ile Glu Leu Ala Gly Phe Pro Ile Leu
625                 630                 635                 640

Ala Gly Val Met Leu Asp Phe Ser Leu Phe Cys Pro Ile Leu Ala Ser
                645                 650                 655

Asn Ser Arg Met Leu Trp Val Pro Ser Ile Cys Ala Ile Trp Pro Pro
            660                 665                 670

Phe Ser Leu Phe Val Tyr Trp Thr Ile Gly Thr Leu Tyr Met Tyr Trp
        675                 680                 685

Phe Ala Lys Tyr Ile Gly Met Ile Arg Lys Asn Ile Ile Arg Pro Gly
    690                 695                 700

Val Leu Phe Phe Ile Arg Ser Pro Glu Asp Pro Asn Ile Lys Ile Leu
705                 710                 715                 720

His Asp Ser Leu Ile His Pro Met Ser Ile Gln Leu Ser Arg Leu Cys
                725                 730                 735

Leu Ser Met Phe Ile Tyr Ala Ile Phe Ile Val Leu Gly Phe Gly Phe
            740                 745                 750

His Thr Arg Ile Phe Phe Pro Phe Met Leu Lys Ser Asn Leu Leu Ser
        755                 760                 765

Val Pro Glu Ala Tyr Lys Pro Thr Ser Ile Ile Ser Trp Lys Phe Asn
    770                 775                 780

Thr Ile Leu Leu Thr Leu Tyr Phe Thr Lys Arg Ile Leu Glu Ser Ser
785                 790                 795                 800

Ser Tyr Val Lys Pro Leu Leu Glu Arg Tyr Trp Lys Thr Ile Phe Lys
                805                 810                 815

Leu Cys Ser Arg Lys Leu Arg Leu Ser Ser Phe Ile Leu Gly Lys Asp
            820                 825                 830

Thr Pro Thr Glu Arg Gly His Ile Val Tyr Arg Asn Leu Phe Tyr Lys
        835                 840                 845

Tyr Ile Ala Ala Lys Asn Ala Glu Trp Ser Asn Gln Glu Leu Phe Thr
    850                 855                 860

Lys Pro Lys Thr Leu Glu Gln Ala Glu Glu Leu Phe Gly Gln Val Arg
865                 870                 875                 880

Asp Val His Ala Tyr Phe Val Pro Asp Gly Val Leu Met Arg Val Pro
                885                 890                 895

Ser Ser Asp Ile Val Ser Arg Asn Tyr Val Gln Thr Met Phe Val Pro
            900                 905                 910

Val Thr Lys Asp Asp Lys Leu Leu Lys Pro Leu Asp Leu Glu Arg Ile
        915                 920                 925

Lys Glu Arg Asn Lys Arg Ala Ala Gly Glu Phe Gly Tyr Leu Asp Glu
    930                 935                 940

Gln Asn Thr Glu Tyr Asp Gln Tyr Tyr Ile Val Tyr Val Pro Pro Asp
945                 950                 955                 960

Phe Arg Leu Arg Tyr Met Thr Leu Leu Gly Leu Val Trp Leu Phe Ala
                965                 970                 975

Ser Ile Leu Met Leu Gly Val Thr Phe Ile Ser Gln Ala Leu Ile Asn
            980                 985                 990

Phe Val Cys Ser Phe Gly Phe Leu Pro Val Val Lys Leu Leu Leu Gly
        995                 1000                1005

Glu Arg Asn Lys Val Tyr Val Ala Trp Lys Glu Leu Ser Asp Ile Ser
    1010                1015                1020

Tyr Ser Tyr Leu Asn Ile Tyr Tyr Val Cys Val Gly Ser Val Cys Leu
1025                1030                1035                1040

Ser Lys Ile Ala Lys Asp Ile Leu His Phe Thr Glu Gly Gln Asn Thr
                1045                1050                1055

Leu Asp Glu His Ala Val Asp Glu Asn Glu Val Glu Glu Val Glu His
            1060                1065                1070

Asp Ile Pro Glu Arg Asp Ile Asn Asn Ala Pro Val Asn Asn Ile Asn
        1075                1080                1085

Asn Val Glu Glu Gly Gln Gly Ile Phe Met Ala Ile Phe Asn Ser Ile
    1090                1095                1100

Phe Asp Ser Met Leu Val Lys Tyr Asn Leu Met Val Phe Ile Ala Ile
1105                1110                1115                1120

Met Ile Ala Val Ile Arg Thr Met Val Ser Trp Val Val Leu Thr Asp
                1125                1130                1135

Gly Ile Leu Ala Cys Tyr Asn Tyr Leu Thr Ile Arg Val Phe Gly Asn
            1140                1145                1150

Ser Ser Tyr Thr Ile Gly Asn Ser Lys Trp Phe Lys Tyr Asp Glu Ser
        1155                1160                1165

Leu Leu Phe Val Val Trp Ile Ile Ser Ser Met Val Asn Phe Gly Thr
    1170                1175                1180

Gly Tyr Lys Ser Leu Lys Leu Phe Phe Arg Asn Arg Asn Thr Ser Lys
1185                1190                1195                1200

Leu Asn Phe Leu Lys Thr Met Ala Leu Glu Leu Phe Lys Gln Gly Phe
                1205                1210                1215

Leu His Met Val Ile Tyr Val Leu Pro Ile Ile Ile Leu Ser Leu Val
            1220                1225                1230

Phe Leu Arg Asp Val Ser Thr Lys Gln Ile Ile Asp Ile Ser His Gly
        1235                1240                1245

Ser Arg Ser Phe Thr Leu Ser Leu Asn Glu Ser Phe Pro Thr Trp Thr
    1250                1255                1260

Arg Met Gln Asp Ile Tyr Phe Gly Leu Leu Ile Ala Leu Glu Ser Phe
1265                1270                1275                1280

Thr Phe Phe Phe Gln Ala Thr Val Leu Phe Ile Gln Trp Phe Lys Ser
                1285                1290                1295

Thr Val Gln Asn Val Lys Asp Glu Val Tyr Thr Lys Gly Arg Ala Leu
            1300                1305                1310

Glu Asn Leu Pro Asp Glu Ser
        1315

<210>    17
<211>    278
<212>    PRT
<213>    Artificial Sequence

<220>
<223>    E3 MARCH5

<400>    17
Met Pro Asp Gln Ala Leu Gln Gln Met Leu Asp Arg Ser Cys Trp Val
1               5                   10                  15

Cys Phe Ala Thr Asp Glu Asp Asp Arg Thr Ala Glu Trp Val Arg Pro
            20                  25                  30

Cys Arg Cys Arg Gly Ser Thr Lys Trp Val His Gln Ala Cys Leu Gln
        35                  40                  45

Arg Trp Val Asp Glu Lys Gln Arg Gly Asn Ser Thr Ala Arg Val Ala
    50                  55                  60

Cys Pro Gln Cys Asn Ala Glu Tyr Leu Ile Val Phe Pro Lys Leu Gly
65                  70                  75                  80

Pro Val Val Tyr Val Leu Asp Leu Ala Asp Arg Leu Ile Ser Lys Ala
                85                  90                  95

Cys Pro Phe Ala Ala Ala Gly Ile Met Val Gly Ser Ile Tyr Trp Thr
            100                 105                 110

Ala Val Thr Tyr Gly Ala Val Thr Val Met Gln Val Val Gly His Lys
        115                 120                 125

Glu Gly Leu Asp Val Met Glu Arg Ala Asp Pro Leu Phe Leu Leu Ile
    130                 135                 140

Gly Leu Pro Thr Ile Pro Val Met Leu Ile Leu Gly Lys Met Ile Arg
145                 150                 155                 160

Trp Glu Asp Tyr Val Leu Arg Leu Trp Arg Lys Tyr Ser Asn Lys Leu
                165                 170                 175

Gln Ile Leu Asn Ser Ile Phe Pro Gly Ile Gly Cys Pro Val Pro Arg
            180                 185                 190

Ile Pro Ala Glu Ala Asn Pro Leu Ala Asp His Val Ser Ala Thr Arg
        195                 200                 205

Ile Leu Cys Gly Ala Leu Val Phe Pro Thr Ile Ala Thr Ile Val Gly
    210                 215                 220

Lys Leu Met Phe Ser Ser Val Asn Ser Asn Leu Gln Arg Thr Ile Leu
225                 230                 235                 240

Gly Gly Ile Ala Phe Val Ala Ile Lys Gly Ala Phe Lys Val Tyr Phe
                245                 250                 255

Lys Gln Gln Gln Tyr Leu Arg Gln Ala His Arg Lys Ile Leu Asn Tyr

260                 265                 270

Pro Glu Gln Glu Glu Ala
        275



<210>    18
<211>    191
<212>    PRT
<213>    Artificial Sequence

<220>
<223>    Artificial Sequence



<400>    18



<210>    19
<211>    96
<212>    PRT
<213>    Artificial Sequence

<220>
<223>    Artificial Sequence

<400>    19
Ser Asp Gln Glu Ala Lys Pro Ser Thr Glu Asp Leu Gly Asp Lys Lys
1               5                   10                  15

Glu Gly Glu Tyr Ile Lys Leu Lys Val Ile Gly Gln Asp Ser Ser Glu
            20                  25                  30

Ile His Phe Lys Val Lys Met Thr Thr His Leu Lys Lys Leu Lys Glu
        35                  40                  45

Ser Tyr Cys Gln Arg Gln Gly Val Pro Met Asn Ser Leu Arg Phe Leu
    50                  55                  60

Phe Glu Gly Gln Arg Ile Ala Asp Asn His Thr Pro Lys Glu Leu Gly
65                  70                  75                  80

Met Glu Glu Glu Asp Val Ile Glu Val Tyr Gln Glu Gln Thr Gly Gly
                85                  90                  95

<210>    20
<211>    371
<212>    PRT
<213>    Artificial Sequence

<220>
<223>    Artificial Sequence

<400>    20
Met Gly His His His His His His Ser Asp Gln Glu Ala Lys Pro Ser
1               5                   10                  15

Thr Glu Asp Leu Gly Asp Lys Lys Glu Gly Glu Tyr Ile Lys Leu Lys
            20                  25                  30

Val Ile Gly Gln Asp Ser Ser Glu Ile His Phe Lys Val Lys Met Thr
        35                  40                  45

Thr His Leu Lys Lys Leu Lys Glu Ser Tyr Cys Gln Arg Gln Gly Val
    50                  55                  60

Pro Met Asn Ser Leu Arg Phe Leu Phe Glu Gly Gln Arg Ile Ala Asp
65                  70                  75                  80

Asn His Thr Pro Lys Glu Leu Gly Met Glu Glu Glu Asp Val Ile Glu
                85                  90                  95

Val Tyr Gln Glu Gln Thr Gly Gly Phe Pro Thr Ile Pro Leu Ser Arg
            100                 105                 110

Leu Phe Asp Asn Ala Met Leu Arg Ala His Arg Leu His Gln Leu Ala
        115                 120                 125

Phe Asp Thr Tyr Gln Glu Phe Glu Glu Ala Tyr Ile Pro Lys Glu Gln
    130                 135                 140

Lys Tyr Ser Phe Leu Gln Asn Pro Gln Thr Ser Leu Cys Phe Ser Glu
145                 150                 155                 160

Ser Ile Pro Thr Pro Ser Asn Arg Glu Glu Thr Gln Gln Lys Ser Asn
                165                 170                 175

Leu Glu Leu Leu Arg Ile Ser Leu Leu Leu Ile Gln Ser Trp Leu Glu
                180                 185                 190

Pro Val Gln Phe Leu Arg Ser Val Phe Ala Asn Ser Leu Val Tyr Gly
            195                 200                 205

Ala Ser Asp Ser Asn Val Tyr Asp Leu Leu Lys Asp Leu Glu Glu Gly
    210                 215                 220

Ile Gln Thr Leu Met Gly Arg Leu Glu Asp Gly Ser Pro Arg Thr Gly
225                 230                 235                 240

Gln Ile Phe Lys Gln Thr Tyr Ser Lys Phe Asp Thr Asn Ser His Asn
                245                 250                 255

Asp Asp Ala Leu Leu Lys Asn Tyr Gly Leu Leu Tyr Cys Phe Arg Lys
                260                 265                 270

Asp Met Asp Lys Val Glu Thr Phe Leu Arg Ile Val Gln Cys Arg Ser
            275                 280                 285

Val Glu Gly Ser Cys Gly Phe Met Gln Ile Phe Val Arg Thr Leu Thr
    290                 295                 300

Gly Arg Thr Ile Thr Leu Glu Val Glu Pro Ser Asp Thr Ile Glu Asn
305                 310                 315                 320

Val Arg Ala Arg Ile Gln Asp Arg Glu Gly Ile Pro Pro Asp Gln Gln
                325                 330                 335

Arg Leu Ile Phe Ala Gly Arg Gln Leu Glu Asp Gly Arg Thr Leu Ser
                340                 345                 350

Asp Tyr Asn Ile Gln Arg Glu Ser Thr Leu His Leu Val Leu Arg Leu
            355                 360                 365

Arg Gly Gly
    370


<210>    21
<211>    371
<212>    PRT
<213>    Artificial Sequence

<220>
<223>    Artificial Sequence


<400>    21
Met Gly His His His His His His Ser Asp Gln Glu Ala Lys Pro Ser
 1               5                  10                  15

Thr Glu Asp Leu Gly Asp Lys Lys Glu Gly Glu Tyr Ile Lys Leu Lys
                20                  25                  30

Val Ile Gly Gln Asp Ser Ser Glu Ile His Phe Lys Val Lys Met Thr
         35                  40                  45

Thr His Leu Lys Lys Leu Lys Glu Ser Tyr Cys Gln Arg Gln Gly Val
     50                  55                  60

Pro Met Asn Ser Leu Arg Phe Leu Phe Glu Gly Gln Arg Ile Ala Asp
65                  70                  75                  80

Asn His Thr Pro Lys Glu Leu Gly Met Glu Glu Glu Asp Val Ile Glu
                85                  90                  95

Val Tyr Gln Glu Gln Thr Gly Gly Phe Pro Thr Ile Pro Leu Ser Arg
            100                 105                 110

Leu Phe Asp Asn Ala Met Leu Arg Ala His Arg Leu His Gln Leu Ala
        115                 120                 125

Phe Asp Thr Tyr Gln Glu Phe Glu Glu Ala Tyr Ile Pro Lys Glu Gln
    130                 135                 140

Lys Tyr Ser Phe Leu Gln Asn Pro Gln Thr Ser Leu Cys Phe Ser Glu
145                 150                 155                 160

Ser Ile Pro Thr Pro Ser Asn Arg Glu Glu Thr Gln Gln Lys Ser Asn
                165                 170                 175

Leu Glu Leu Leu Arg Ile Ser Leu Leu Leu Ile Gln Ser Trp Leu Glu
            180                 185                 190

Pro Val Gln Phe Leu Arg Ser Val Phe Ala Asn Ser Leu Val Tyr Gly
        195                 200                 205

Ala Ser Asp Ser Asn Val Tyr Asp Leu Leu Lys Asp Leu Glu Glu Gly
    210                 215                 220

Ile Gln Thr Leu Met Gly Arg Leu Glu Asp Gly Ser Pro Arg Thr Gly
225                 230                 235                 240

Gln Ile Phe Lys Gln Thr Tyr Ser Lys Phe Asp Thr Asn Ser His Asn
                245                 250                 255

Asp Asp Ala Leu Leu Lys Asn Tyr Gly Leu Leu Tyr Cys Phe Arg Lys
            260                 265                 270

Asp Met Asp Lys Val Glu Thr Phe Leu Arg Ile Val Gln Cys Arg Ser
        275                 280                 285

Val Glu Gly Ser Cys Gly Phe Met Gln Ile Phe Val Arg Thr Leu Thr
    290                 295                 300

Gly Arg Thr Ile Thr Leu Glu Val Glu Pro Ser Asp Thr Ile Glu Asn
305                 310                 315                 320

Val Arg Ala Arg Ile Gln Asp Arg Glu Gly Ile Pro Pro Asp Gln Gln
                325                 330                 335

Arg Leu Ile Phe Ala Gly Arg Gln Leu Glu Asp Gly Arg Thr Leu Ser
            340                 345                 350

Asp Tyr Asn Ile Gln Lys Glu Ser Thr Leu His Leu Val Leu Arg Pro
        355                 360                 365

Arg Gly Gly
    370


<210>    22
<211>    268

<212>    PRT
<213>    Artificial Sequence

<220>
<223>    Artificial Sequence


<400>    22
Met Gln Ile Phe Val Arg Thr Leu Thr Gly Arg Thr Ile Thr Leu Glu
1               5                   10                  15

Val Glu Pro Ser Asp Thr Ile Glu Asn Val Arg Ala Arg Ile Gln Asp
            20                  25                  30

Arg Glu Gly Ile Pro Pro Asp Gln Gln Arg Leu Ile Phe Ala Gly Arg
        35                  40                  45

Gln Leu Glu Asp Gly Arg Thr Leu Ser Asp Tyr Asn Ile Gln Lys Glu
    50                  55                  60

Ser Thr Leu His Leu Val Leu Arg Pro Arg Gly Gly Asp Phe Pro Thr
65                  70                  75                  80

Ile Pro Leu Ser Arg Leu Phe Asp Asn Ala Met Leu Arg Ala His Arg
                85                  90                  95

Leu His Gln Leu Ala Phe Asp Thr Tyr Gln Glu Phe Glu Glu Ala Tyr
            100                 105                 110

Ile Pro Lys Glu Gln Lys Tyr Ser Phe Leu Gln Asn Pro Gln Thr Ser
        115                 120                 125

Leu Cys Phe Ser Glu Ser Ile Pro Thr Pro Ser Asn Arg Glu Glu Thr
    130                 135                 140

Gln Gln Lys Ser Asn Leu Glu Leu Leu Arg Ile Ser Leu Leu Leu Ile
145                 150                 155                 160

Gln Ser Trp Leu Glu Pro Val Gln Phe Leu Arg Ser Val Phe Ala Asn
                165                 170                 175

Ser Leu Val Tyr Gly Ala Ser Asp Ser Asn Val Tyr Asp Leu Leu Lys
            180                 185                 190

Asp Leu Glu Glu Gly Ile Gln Thr Leu Met Gly Arg Leu Glu Asp Gly
        195                 200                 205

Ser Pro Arg Thr Gly Gln Ile Phe Lys Gln Thr Tyr Ser Lys Phe Asp
    210                 215                 220

Thr Asn Ser His Asn Asp Asp Ala Leu Leu Lys Asn Tyr Gly Leu Leu
225                 230                 235                 240

Tyr Cys Phe Arg Lys Asp Met Asp Lys Val Glu Thr Phe Leu Arg Ile
                245                 250                 255

Val Gln Cys Arg Ser Val Glu Gly Ser Cys Gly Phe
            260                 265


<210>    23
<211>    372
<212>    PRT

<213>    Artificial Sequence

<220>
<223>    Artificial Sequence


<400>    23
Met Gly His His His His His His Ser Asp Gln Glu Ala Lys Pro Ser
1               5                   10                  15

Thr Glu Asp Leu Gly Asp Lys Lys Glu Gly Glu Tyr Ile Lys Leu Lys
            20                  25                  30

Val Ile Gly Gln Asp Ser Ser Glu Ile His Phe Lys Val Lys Met Thr
        35                  40                  45

Thr His Leu Lys Lys Leu Lys Glu Ser Tyr Cys Gln Arg Gln Gly Val
    50                  55                  60

Pro Met Asn Ser Leu Arg Phe Leu Phe Glu Gly Gln Arg Ile Ala Asp
65                  70                  75                  80

Asn His Thr Pro Lys Glu Leu Gly Met Glu Glu Glu Asp Val Ile Glu
                85                  90                  95

Val Tyr Gln Glu Gln Thr Gly Gly Met Gln Ile Phe Val Arg Thr Leu
            100                 105                 110

Thr Gly Arg Thr Ile Thr Leu Glu Val Glu Pro Ser Asp Thr Ile Glu
        115                 120                 125

Asn Val Arg Ala Arg Ile Gln Asp Arg Glu Gly Ile Pro Pro Asp Gln
    130                 135                 140

Gln Arg Leu Ile Phe Ala Gly Lys Gln Leu Glu Asp Gly Arg Thr Leu
145                 150                 155                 160

Ser Asp Tyr Asn Ile Gln Arg Glu Ser Thr Leu His Leu Val Leu Arg
                165                 170                 175

Pro Arg Gly Gly Asp Phe Pro Thr Ile Pro Leu Ser Arg Leu Phe Asp
            180                 185                 190

Asn Ala Met Leu Arg Ala His Arg Leu His Gln Leu Ala Phe Asp Thr
        195                 200                 205

Tyr Gln Glu Phe Glu Glu Ala Tyr Ile Pro Lys Glu Gln Lys Tyr Ser
    210                 215                 220

Phe Leu Gln Asn Pro Gln Thr Ser Leu Cys Phe Ser Glu Ser Ile Pro
225                 230                 235                 240

Thr Pro Ser Asn Arg Glu Glu Thr Gln Gln Lys Ser Asn Leu Glu Leu
                245                 250                 255

Leu Arg Ile Ser Leu Leu Leu Ile Gln Ser Trp Leu Glu Pro Val Gln
            260                 265                 270

Phe Leu Arg Ser Val Phe Ala Asn Ser Leu Val Tyr Gly Ala Ser Asp
        275                 280                 285

Ser Asn Val Tyr Asp Leu Leu Lys Asp Leu Glu Glu Gly Ile Gln Thr
    290                 295                 300

Leu Met Gly Arg Leu Glu Asp Gly Ser Pro Arg Thr Gly Gln Ile Phe
305                 310                 315                 320

Lys Gln Thr Tyr Ser Lys Phe Asp Thr Asn Ser His Asn Asp Asp Ala
                325                 330                 335

Leu Leu Lys Asn Tyr Gly Leu Leu Tyr Cys Phe Arg Lys Asp Met Asp
            340                 345                 350

Lys Val Glu Thr Phe Leu Arg Ile Val Gln Cys Arg Ser Val Glu Gly
        355                 360                 365

Ser Cys Gly Phe
    370